# EUROPEAN PATENT APPLICATION

(11) **EP 4 454 757 A1**
(43) Date of publication of application: **30.10.2024**
(21) Application number: 23190482.2
(22) Date of filing: 09.08.2023
(51) Int. Cl.: B01L 3/00, A61B 10/00, G01N 1/02

(54) **DEVICE FOR TESTING ANALYTE IN LIQUID SAMPLE**

(30) Priority: 26.04.2023 CN 202310472844; 09.05.2023 US 202363501047 P
(71) Applicant: Hangzhou Biotest Biotech Co., Ltd., Yuhang District Hangzhou (CN)
(72) Inventor: WU, Shujiang, Hangzhou (CN); HONG, Liang, Hangzhou (CN); ZHAO, Qihui, Hangzhou (CN); WU, Junsheng, Hangzhou (CN); WANG, Yang, Hangzhou (CN); ZENG, Xiuying, Hangzhou (CN)
(74) Representative: Piotrowicz, Pawel Jan Andrzej

(57) **Abstract**

The present invention provides a device for testing an analyte in a liquid sample, and the device includes: a detection chamber, where the detection chamber is configured to receive a testing element, and the testing element is configured to test the analyte in the liquid sample; and a collector, where an absorption element on the collector is configured to absorb the liquid sample, and the collector is accommodated in the test device. Such device can be used to achieve liquid self-test, for example, collection and self-test of a saliva sample.

## Description

### CROSS-REFERENCE TO RELATED APPLICATIONS

This application claims priority to Chinese Patent Application No.202310472844.5, filed on April 26, 2023 and U.S. Provisional Application No. 63/501,047, filed on May 9, 2023, and the specification, abstract, accompanying drawings, and claims of this application are made a part of this application.

### BACKGROUND OF THE INVENTION

### Field of the Invention

The present invention relates to a device for collecting a liquid sample, in particular, a device for collecting and testing an analyte in a liquid sample in the field of rapid diagnosis, such as a urine and saliva collection and test device.

### Description of the Related Art

The following description is merely an introduction of some background general knowledge and does not constitute any limitation to the present invention.

At present, the test device for detecting the presence or absence of an analyte in sample is widely used in hospitals or homes, and such test device for rapid diagnosis includes one or more test strips, such as early pregnancy detection and drug abuse detection. Such test device for rapid diagnosis is very convenient, and can obtain a testing result from the test strips after one minute or no at most about ten minutes. Drug test is widely used by the drug control department, the Public Security Bureau, drug rehabilitation centers, physical examination centers, physical examination offices of national conscription, etc. The drug test is diverse and frequent. Some detections are required to collect samples and then samples are detected in professional testing agencies or testing laboratories, and some detections need to be completed in the site in time, for example, persons who drive after drug use need to be tested on the spot (referred to as "Drug Driving"), to obtain the testing results in time.

In conventional detection, it is particularly desired that micro-samples can be accurately tested, such that one or more indicators can be accomplished only with a small number of samples without deliberately pursuing the volume or number of samples. For example, U.S. Patent Application US14/383,149 discloses a micro-sample, in which the sample can be obtained and fitted to a test device. However, the device requires an additional buffer to rinse the micro-sample from the collector, and furthermore, the micro-sample is in direct contact with the label, which allows the micro-sample collector to be in contact with a label area and affects the chemical properties of the label. A sample collector is also described in EP2507632B1 and compressed by an additional pressing structure; although the sample collector is improved, its operation is not simple, the sample sampler is inserted into an area and then the collector is compressed by an additional step, the addition step is equivalent to two steps, and the collector is stored separately, which still has some drawbacks.

In view of the above technical problems, it is necessary to improve them and provide an alternative approach to solve the drawbacks of the prior art, especially for a drug self-test device or method, and collection and testing of micro-samples.

### BRIEF SUMMARY OF THE INVENTION

On the one hand, the present invention provides a test device, and the test device includes a detection chamber, where the detection chamber is configured to receive a testing element, and the testing element is configured to test an analyte in a liquid sample; the test device further includes a sample collector, and the sample collector includes an absorption element configured to absorb the liquid sample, where an accommodating chamber is provided on the test device and configured to accommodate the absorption element of the sample collector. The collector and the test device are integrated to reduce the packaging cost.

In some embodiments, the detection chamber includes the testing element, and the testing element includes a sample application area, a label area located downstream of the sample application area, and a testing or detection area located downstream the label area.

In some embodiments, the accommodating chamber for accommodating the absorption element of the collector can be located in or on the accommodating area of the test device, and the accommodating chamber includes the sample application area of the testing element. In some embodiments, the sample application area is located at a bottom of the accommodating chamber.

In some embodiments, the absorption element of the sample collector has a first state and a second state in the accommodating chamber; with the absorption element of the collector being in the first state, the absorption element is not in contact with the sample application area. In some embodiments, the accommodating chamber is used to accommodate the absorption element. In some embodiments, with the absorption element of the collector being in the second state, the absorption element includes no liquid sample or absorbs no liquid sample. Alternatively, the absorption element of the collector is not in direct contact with the sample application area when not tested; and the absorption element absorbs the liquid sample when required to be tested. When the absorption element absorbing the liquid sample is located in the accommodating chamber again, the test is started. For example, the absorption element is compressed to release the liquid to the sample application area, thus starting the test. In some embodiments, when the absorption element is in the accommodating chamber again or in the second state, the absorption element absorbs the liquid sample; when the absorption element is in a process of entering the accommodating chamber, the absorption element is compressed, such that the liquid from the absorption element is released to the sample application area, thereby automatically starting the test. Such automatic compression means that when the absorption element is in the process of entering the accommodating chamber, the absorption element interacts with the accommodating chamber, and force generated by such interaction causes the absorption element to be compressed, thus releasing the liquid sample to the sample application area. Force generated by interaction herein can be pressure or extrusion pressure, and is directly or indirectly applied to the absorption element, such that the absorption element is compressed by the force, such as the pressure or the extrusion pressure, which can also be called "force that causes the absorption element to be compressed". So-called compression means bearing force by the absorption element under a natural condition, making the absorption element shrink and reduce its volume. In some embodiments, the absorption element needs to depart from the accommodating chamber in the first state from the accommodating chamber, collects the liquid sample, and then enters the accommodating chamber again after collecting the liquid sample; and with the absorption element being in the second state, the absorption element is compressed in the process of entering the second state.

In some embodiments, with the absorption element of the collector being in the second state, the absorption element is in contact with the sample application area. Thus, the absorption element has the liquid sample. In some embodiments, with the absorption element of the collector being in the second state, the absorption element interacts with the accommodating chamber, such that the absorption element is compressed. Such interaction means that when the absorption element is configured or placed in the accommodating chamber, the absorption element and the accommodating chamber coordinate, fit or interact with each other to generate a force causing the absorption element to be compressed. In some embodiments, a carrier with the absorption element and the accommodating chamber coordinate, fit or interact with each other to generate a force, such that the absorption element is compressed, thereby releasing the liquid sample from the absorption element. For example, the absorption element is compressed by the carrier element, or the absorption element being positioned between the sample application area and the carrier leads to interaction to generate a force that causes the absorption element to be compressed. In such a way, the absorption element may be fixed directly to the carrier, for example a rigid plastic carrier. Therefore, these absorption elements are made of compressed materials, and such materials can also be water-absorbent and can absorb water and make the absorbed water stay thereon. When the absorption elements are compressed, water or liquid samples can be extruded out. As disclosed above, the so-called compression means volume reduction, and the volume reduction causes liquid to be released. In some embodiments, the absorption element is loaded on a carrier; the carrier has an insertion structure and the accommodating chamber has an accommodating structure, for example, a jack. When the insertion structure on the carrier is inserted into the accommodating structure of the accommodating structure, a part of the absorption element is in contact with the sample application area, and the absorption element can be compressed during the insertion. Such interaction herein means that the insertion structure of the carrier and the accommodating structure of the accommodating chamber interact to generate a force.

In some embodiments, when the insertion structure is inserted into the accommodating structure, the entire absorption element covers the sample application area such that the absorption element is compressed in the process, releasing a part of the liquid sample onto the sample application area. In some embodiments, the carrier with the absorption element is obliquely inserted into the accommodating structure, and then the carrier is parallel to a first accommodating area and located in the accommodating chamber, such that the absorption element is changed from being obliquely inserted into the accommodating structure to covering the accommodating chamber, and the absorption element is compressed due to a reduced distance (relative to the bottom of the accommodating chamber or the sample application area located at the bottom). Alternatively, the carrier with the absorption element obliquely enters the accommodating chamber, a part of the carrier enters the accommodating chamber or is in contact with the sample application area in the accommodating chamber, and then the carrier completely covers the accommodating chamber or is completely in contact with the sample application area in the accommodating chamber. A way to load the absorption element onto the carrier is to adhere to the carrier. In such a way, the absorption element is compressed as soon as the absorption element is configured to enter the accommodating chamber, releasing the liquid sample to the sample application area located in the accommodating chamber, which reduces additional operation. In some embodiments, the interaction of the carrier with the accommodating chamber may also be that the absorption element on the carrier is compressed once the carrier is inserted into the accommodating chamber and when the carrier and the accommodating chamber are at a fixed position. The insertion process may be that a position where the carrier is inserted into the accommodating chamber is fixed. After the insertion, the carrier covers the entire accommodating chamber, and the insertion position of the carrier is fixed in a covering process; the carrier has a function equivalent to a lever, such that the carrier covers the accommodating chamber and the absorption element on the carrier is naturally pressed in the covering process, thereby facilitating releasing the liquid sample. The sample application area in the accommodating chamber is at a fixed position. Although the sample application area is made of water-absorbent materials such as glass fiber, it has very low compressibility, and the carrier on the absorption element has plastic rigidity and cannot be compressed. The compressibility of the absorption element is greater than that of the rigid carrier and the sample application area. When the absorption element is located between the carrier and the sample application area, the carrier applies pressure to the absorption element, and the absorption element is allowed to be compressed under the cooperation of the sample application area and the carrier, thereby releasing the liquid sample to the sample application area. Term "compressibility" herein means that great volume reduction indicates great compressibility when a same force is applied.

In some embodiments, the carrier is connected to a handle, and the handle is accommodated in a docking area of the test device, and the docking area is located upstream of the accommodating chamber. In some embodiments, when the absorption element departs from the accommodating chamber, the handle also departs from together the docking area and is held by the hand, and then the absorption element is allowed to enter the mouth to collect saliva; alternatively, the handle is allowed to depart from the docking area and the absorption element is driven out of the accommodating chamber, for example, the carrier is driven out of the accommodating chamber such that the absorption element also departs from the accommodating chamber together. When the absorption element enters the accommodating chamber, the handle also enters the docking area and is fixed in the docking area; alternatively, the carrier with the absorption element enters the accommodating chamber, and then the handle approaches the docking area, such that the handle also enters the docking area and is fixed in the docking area. The absorption element is loaded on the carrier and obliquely enters the accommodating chamber. Then, the handle also enters the docking area, such that the carrier with the absorption element naturally covers the accommodating chamber, especially the bottom of the accommodating chamber or the sample application area of the testing element located at the bottom of the accommodating chamber, such that the handle is used to apply a pressure to the carrier, the sample at the bottom of the accommodating chamber is an extrusion force between the sample application area (for example, a glass fiber made into the sample application area) and the carrier and the extrusion force is naturally applied to the absorption element, thereby compressing the absorption element and releasing liquid. Of course, in this case, the carrier and the accommodating chamber are laterally fixed (relative to longitudinal left and right positions of the test device) by contacting a structure, and the handle connected with the carrier is similar to a point on the carrier as a pivot in a process from departing from the docking area to entering the docking area, while a point where the carrier is fixed with the accommodating chamber is a movable point (a process where the carrier approaches the bottom of the accommodating chamber therein), and the handle is a point where pressure is applied. The pressure applied to the handle drives the pivot to gradually approach the bottom of the accommodating chamber to apply the pressure to the absorption element on the carrier, such that the absorption element can be compressed through the pressure applied to the absorption element by the carrier, releasing the liquid. In other words, it can be understood that the carrier on the collector, the absorption element on the carrier, and the handle fixedly connected with the carrier apply pressure to the absorption element when the carrier is allowed to enter the accommodating chamber, and the pressure is applied to the absorption element, such that the volume of the absorption element is reduced, releasing the liquid sample on the absorption element. Although the process is relatively fast, it also provides pressure to the absorption element, allowing the absorption element to be compressed to release the liquid sample, thereby starting the test.

In some embodiments, when the absorption element of the collector is in the first state, the collector does not absorb the liquid sample, and at the same time is not in direct contact with the sample application area. In some embodiments, in this state, the absorption element is protected by a cover body, and the cover body is located in the accommodating chamber. Thus, since the cover body protects the absorption element and isolates the absorption element from the sample application area, the absorption element cannot be in direct contact with the sample application area. When liquid needs to be collected, the absorption element is taken out from the cover body and then in contact with the liquid sample, such that the absorption element absorbs the liquid sample. After collecting the liquid, the absorption element enters the accommodating chamber again, thereby starting the test according to the above method.

In some embodiments, the carrier is transparent, a groove is provided thereon, a color bar is provided in the groove, and the color bar is in fluid communication with the absorption element and used to display whether the absorption element has collected a sufficient liquid sample. In some embodiments, the carrier is a plane, and the absorption element is bonded on the plane, and the color bar is in direct contact with the absorption element. In some embodiments, the entire color bar covers the surface of the absorption element. In some embodiments, the carrier is provided with the insertion structure. In some embodiments, the carrier is provided with a slot insertion structure, and the slot insertion structure can be inserted into the slot or the jack of the accommodating chamber. It can be understood that when the slot insertion structure is protected by the cover body or when the absorption element is located in the cover body, the slot insertion structure cannot be inserted into the slot of the accommodating chamber. When the absorption element absorbs the liquid sample and needs to be started for test, the slot insertion structure can be inserted into the slot and the test is started; in this case, the cover body is detached from the carrier.

In some embodiments, the slot insertion structure is obliquely inserted into the slot. In this case, a part of the absorption element may be in contact with the sample application area of the testing element. However, only when the carrier with the absorption element completely enters the accommodating chamber, and the absorption element almost completely enters the accommodating chamber. In this case, the absorption element covers the sample application area. In some embodiments, the handle of the collector is allowed to enter the docking area and is fixed in the docking area due to a combination of the slot insertion structure and the slot, an inclined carrier is allowed to be parallel to the bottom of the accommodating chamber, and the absorption element is allowed to enter the accommodating chamber, or the absorption element and the carrier are all allowed to cover the bottom of the accommodating chamber; in this case, the absorption element is compressed to release a part of the liquid to the sample application area. The above absorption elements are compressed by the pressure applied to the absorption element by the carrier as a result of a combination of the collector and the accommodating chamber, without the help of external force. In other words, such compression is an operation that the collector is returned to the test device again after departing from the test device and collecting the liquid sample, which naturally compresses the absorption element and releases the liquid to the sample application area at the bottom of the accommodating chamber to start the test.

In order to ensure that the absorption element is continuously compressed on the accommodating chamber and continuously releases the liquid sample, it is necessary to continuously apply a pressure to the absorption element, and such pressure applied to the absorption element is called external force. Therefore, in some embodiments, the test device includes a pressing element, and the pressing element can apply pressure to the carrier with the absorption element, such that the pressing element, the absorption element, and the sample application area are vertically arranged. In some embodiments, the pressing element can slide on the test device, and can slide onto and cover the accommodating chamber, thus applying pressure to the absorption element located in the accommodating chamber. In some embodiments, the pressing element is located in a window area for reading a testing result at an initial position, and the window area is located upstream of the sample application area, or the window area is located upstream of the accommodating chamber. Thus, the pressing element initially covers the window area and slides onto the accommodating chamber when required to be tested, which continues to apply a constant pressure to the absorption element. Thus, the pressing element being located in the window area also protects the window area, which prevents the absorption element from entering the window area and causing improper operation. This is because the window area and the accommodating chamber are located proximal to each other. After an operator removes the collector from the accommodating chamber to collect samples and if no pressing element protects or cover the window area, the operator can easily insert the absorption element into the window area, thus damaging the testing area in the window area and failing to complete the test naturally. However, the pressing element slides onto the accommodating chamber to apply pressure to the absorption element while exposing the window area. In this case, the testing result can be read through the window area, and the sliding element located on the accommodating chamber applies a constant pressure to the carrier.

Therefore, in some embodiments, when the absorption element is in the first state, the pressing element is not above the absorption element or on the accommodating chamber; alternatively, in some embodiments, the pressing element is located in the window area for reading a testing result of the test device, and the absorption element is in the first state where no liquid sample is included. When the absorption element is located in the second state, the carrier provides a compressive force to the absorption element, and the pressing element slides from the window area to the downstream accommodating chamber, providing a constant pressure to the carrier.

In some embodiments, the test device includes an upper card and a lower card, where the testing element is located between the upper card and the lower card; an accommodating chamber and a window area are arranged on the upper card; a part of the testing element on the lower card, especially for the sample application area of the testing element, is exposed out of the accommodating chamber, and the testing area of the testing element is exposed out of the window area. When the absorption element is in the first state where no liquid sample is included, the absorption element is located in the accommodating chamber and is not in direct contact with the sample application area, and the pressing element covers the window area. In some embodiments, after the absorption element includes the liquid sample and enters the accommodating chamber, the pressing element slides from the upstream window area to the downstream accommodating chamber.

In some embodiments, the label area is located downstream of the sample application area, so the absorption element is in contact with the sample application area without the label area, instead of being in contact with the label area. In addition, the sample application area includes no antibody or antigen, and only serves to receive the liquid sample released by the absorption element. In some embodiments, the sliding element does not carry any other chemical substances, and only provides a constant pressure to the absorption element, or the sliding element applies the pressure to the carrier to compress the absorption element. The label area is located between the accommodating chamber and the window area for reading a testing result or is hidden.

On the other hand, the present invention provides a method for testing an analyte in a liquid sample, and the method includes:
providing a test device, where the test device includes a detection chamber, the detection chamber is used to receive a testing element and includes the testing element, and the testing element is configured to test the analyte in the liquid sample; and a sample collector, where the collector includes an absorption element, an accommodating area is provided on the test device and used to accommodate the absorption element, the absorption element has a first state and a second state in the accommodating chamber; the absorption element of the collector is allowed to be in the first state and is not allowed to absorb the liquid sample; and the absorption element of the collector is allowed to be in the second state and absorbs the liquid sample.

In some embodiments, the accommodating chamber includes a sample application area of a testing element.

In some embodiments, the absorption element is allowed to depart from the accommodating chamber, absorb the liquid sample, and then enter the accommodating chamber and be in the second state.

In some embodiments, with the absorption element of the sample collector being in the second state, pressure is applied to the absorption element to release the liquid sample; alternatively, with the absorption element of the sample collector being in the second state, the absorption element is compressed to release the liquid sample. In some embodiments, the pressure applied to the absorption element is the pressure applied to the absorption element through interaction between the collector and the accommodating area when the absorption element enters the accommodating chamber.

In some embodiments, the absorption element is loaded on a carrier, such that the carrier fits with the accommodating chamber, applying pressure to the absorption element, or applying pressure to the carrier, thereby allowing the carrier to apply the pressure to the absorption element. In this way, the absorption element can be compressed. In some embodiments, the accommodating chamber includes the sample application area of the testing element, and the absorption element is allowed to be located between the carrier and the sample application area. When the carrier fits with the accommodating chamber, the carrier applies a pressure to the absorption element, thereby compressing the absorption element.

In some embodiments, the carrier includes an insertion structure, and the accommodating chamber includes a slot structure, such that the absorption element is introduced into the accommodating chamber by the carrier when the insertion structure is inserted into the slot structure. In some embodiments, the carrier obliquely enters the accommodating chamber, then is parallel to the sample application area of the testing element in the accommodating chamber, and is allowed to cover the sample application area, such that the absorption element covers the sample application area, and the carrier applies pressure to the absorption element, allowing the absorption element to be compressed.

In some embodiments, a handle integrally connected with the carrier is located in the docking area of the test device, and the docking area is located upstream of the accommodating chamber of the absorption element. The carrier with the absorption element is allowed to be obliquely inserted into the slot of the accommodating chamber, and the handle is also allowed to enter the docking area and is fixed therein, such that a pressure is applied to the carrier through the handle to squeeze the absorption element.

In some embodiments, after the handle is fixed in the docking area, or after the absorption element is located in the accommodating chamber, the pressing element located on the window area for reading a testing result slides into the accommodating chamber, and the pressure is continuously applied to the absorption element, thereby continuously releasing the liquid sample.

In some embodiments, the absorption element is allowed to be in the first state, and the absorption element is not allowed to be in direct contact with the sample application area of the testing element. In some embodiments, the absorption element is allowed to be located in a cover body, and the cover body is allowed to be located in the accommodating chamber. In some embodiments, the cover body is allowed to prevent the pressing element located in the window area for reading a testing result from sliding, for example, sliding to the downstream accommodating chamber.

In some embodiments, the accommodating chamber includes a sample application area of a testing element. In some embodiments, the sample application area is located upstream of the label area, and the label area is located upstream of a testing result area.

In some embodiments, the docking area for accommodating the handle of the collector is located upstream of the accommodating chamber for accommodating the absorption element, and the accommodating chamber for accommodating the absorption element is located upstream of a testing result display area or window.

In some embodiments, the absorption element is located on one carrier and the carrier includes an indicator strip that is in fluid communication with the absorption element; and when the absorption element absorbs the liquid sample, the indicator strip is used for indicating whether the absorption element has fully absorbed the liquid sample. In some embodiments, the absorption element extends into the mouth to collect a saliva sample.

According to another aspect of the present invention, the present invention provides a liquid sample collector, and the collector includes a handle and a carrier connected with the handle, and an absorption element capable to be compressed is fixed onto the carrier, where the absorption element is configured to absorb a liquid sample, and the carrier includes an indicator strip for indicating whether the absorption element absorbs a sufficient liquid sample, and the indicator strip is in fluid communication with the absorption element. In an embodiment, the entire indicator strip covers the absorption element.

In some embodiments, the carrier is transparent, and a groove is arranged on a side where the absorption element is loaded, and the indicator strip is in the groove; and when the absorption element is loaded on the side, the entire absorption element covers the absorption element. In some embodiments, the absorption element has a front face and a back face, the surface of the carrier is generally coated with glue, the back face of the absorption element is bonded to the carrier by the glue, and the entire indicator strip covers the back face of the absorption element. The indicator strip includes a reagent that changes color when contacting water, and is colorless if not relieved.

In some embodiments, the carrier with the absorption element is located in one cover body. In some embodiments, a through hole connected with the handle has a specified thickness, on which the carrier is embedded; and the absorption element is bonded to a side of the carrier. In some embodiments, the carrier is provided with a through hole through which the indicator strip passes to be in fluid communication with the absorption element. In some embodiments, a side of the carrier is provided with a groove, the indicator strip is located in the groove, and at the same time, the absorption element is bonded to the carrier through a cover surface. In some embodiments, the indicator strip displays red or pink when contacting liquid, and is white or colorless when being dried. In some embodiments, the carrier is transparent.

In some embodiments, the absorption element can absorb liquid samples within 0.1-5 microlitres. In some embodiments, the absorption element is the material described in the present invention patent EP22185144.7 previously applied by the Applicant.

In all the above embodiments, the compressibility of the absorption element is greater than that of the sample application area of the testing element, that is, under a same pressure, the deformation degree of the absorption element is greater than that of the sample application area, for example, the shrinkage degree of the absorption element is greater than or far greater than that of the sample application area. For example, a length of the absorption element is 4-5 millimeters and a thickness of the sample application area is 2 millimeters; under the same pressure, the absorption element is compressed to a thickness of 1 millimeter, while the sample application area is kept at 2 millimeters, or about 1.80-1.90 millimeters, and is hardly compressed. This could be called an indication of the compression degree. Generally, the carrier with the absorption element is rigid and incompressible, and the sample application area is also made of absorbent material. However, relative to the absorption element, under a same force, the extrusion degree of the absorption element is much more than an absorbent material in the sample application area. Generally, the absorption element is a sponge, and polyester fiber and absorbent material are fiberglass or filter paper; in addition, the sample application area is located on a rigid lower card. Thus, when the carrier applies pressure to the absorption element, the sample application area of the testing element located on the rigid lower card and the absorption element are located between the carrier and the lower card, such that the compressibility of the absorption element is greater than that of the sample application area, the absorption element is more likely to be compressed, and the liquid sample is released onto the absorption element.

### Beneficial effect

The above structure can be used for testing an analyte in a liquid sample, especially for sampling of oral samples. The structure just scraps an oral cavity from a collector or slightly scrapes it, so that the absorption element can absorb saliva in the oral cavity. The structure has the advantages of simple sampling and convenient operation, and can achieve self-test or POCT (point-of-care testing) through a professional structure.

### BRIEF DESCRIPTION OF THE DRAWINGS

FIG. 1 is a schematic diagram showing a three-dimensional structure of an exploded structure of a test device according to a specific embodiment of the present invention.
FIG. 2 is a structural schematic diagram of a combination state according to a specific embodiment of the present invention, where an absorption element of a collector is in a first state.
FIG. 3 is a schematic diagram of an exploded structure of an upper card and a lower card of a test device according to a specific embodiment of the present invention, where a testing element is located between the upper card and the lower card, and optionally, the testing element is located on the lower card.
FIG. 4 is a schematic diagram showing a three-dimensional structure of a back face of an upper card according to a specific embodiment of the present invention.
FIG. 5 is a schematic diagram showing a three-dimensional structure of a cover covering a collector according to a specific embodiment of the present invention.
FIG. 6 is a schematic diagram showing a three-dimensional structure of a collector according to a specific embodiment of the present invention, where no absorption element is loaded on the carrier.
FIG. 7 is a schematic diagram showing a formal structure of a collector according to a specific embodiment of the present invention.
FIG. 8 is a schematic diagram showing an exploded structure of a collector and an absorption element according to a specific embodiment of the present invention.
FIG. 9 is a schematic diagram showing an exploded structure of a collector according to a specific embodiment of the present invention.
FIG. 10 is a schematic diagram showing a longitudinal cross-section structure of a combination of a test device and a collector according to a specific embodiment of the present invention.
FIG. 11 is a partially enlarged schematic diagram of a cross-section diagram.
FIG. 12A is a schematic structural diagram of an operation process of a collector entering an accommodating chamber according to a specific embodiment of the present invention (the collector is inserted into a first accommodating chamber).
FIG. 12B is a schematic structural diagram of an operation process of a collector entering an accommodating chamber according to a specific embodiment of the present invention (the collector is entirely located in a first accommodating chamber).
FIG. 12C is a schematic structural diagram of an operation process of a collector entering an accommodating chamber according to a specific embodiment of the present invention (the collector is inserted into a first accommodating chamber).
FIG. 12D is a schematic structural diagram of an operation process of a collector entering an accommodating chamber according to a specific embodiment of the present invention (the collector is entirely located in a first accommodating chamber).
FIG. 13 is a schematic diagram of a combined structure of a test device and a collector according to a specific embodiment of the present invention, and also a schematic diagram of an absorption element of the collector in a second state, and in this case that a pressing element covers a window area for reading a testing result.
FIG. 14 shows that an absorption element of a collector is located in an accommodating chamber according to a specific embodiment of the present invention, and a pressing element slides from a window area for reading a testing result to an accommodating area to continuously apply pressure to an absorption element while exposing the window area for reading a testing result for reading a subsequent result.
FIG. 15 shows that in absence of a sliding element, an absorption element covers an accommodating chamber and is compressed in a second state according to another embodiment of the present invention; in the second state, a structure of a collector fits with a structure of the accommodating chamber; and when the absorption element enters into the accommodating chamber, the absorption element is in contact with the sample application area in this area and releases a liquid sample.
FIG. 16 is an exploded schematic diagram of a combined structure of a test device and a collector according to another specific embodiment of the present invention.
FIG. 17 is a schematic diagram of a combined structure of a test device and a collector according to another specific embodiment of the present invention, where an absorption element of the collector is protected by a cover body and located in an accommodating chamber of the test device, and in this case that the absorption element is not in contact with a sample application area of a testing element (in absence of a pressing element).
FIG. 18 is a schematic diagram of a collection structure of a collector (an absorption element is protected by a cover body).
FIG. 19 is an exploded diagram of a structure of a collector.
FIG. 20 is a schematic structural diagram of a test device (without a collector).
FIG. 21 is a schematic structural diagram showing that a test device fits with a collector so that an absorption element on the collector is located in an accommodating chamber and is in contact with a sample application area at a bottom of the accommodating chamber.
FIG. 22 is a top view of a testing element according to a specific embodiment of the present invention.
FIG. 23 is a schematic diagram showing a three-dimensional structure of a testing element according to a specific embodiment of the present invention.

### DETAILED DESCRIPTION OF THE INVENTION

The following further describes the structures involved in the present invention or the technical terms used therein. Unless otherwise specified, they shall be understood and explained according to the general terms commonly used in the prior art.

### Detection

Detection means assaying or testing presence or absence of a substance or material, including but not limited to, chemical substance, organic compound, inorganic compound, metabolite, drug, drug metabolite, organic tissue, metabolite of organic tissue, nucleic acid, protein or polymer. In addition, detection means that the amount of a substance or material is tested. Further, assay also means immunoassay, chemical assay, enzyme assay, and the like.

### Sample

The samples detected or collected by the test device of the present invention include biological liquid (for example, case liquid or clinical sample). Liquid samples or liquid specimens, or fluid specimens or fluid samples may be derived from solid or semi-solid samples, including feces, biological tissues and food samples. The solid or semi-solid samples may be converted to liquid samples by any appropriate methods, such as mixing, mashing, macerating, incubating, dissolving, or digesting the solid samples by enzymolysis in suitable solutions, such as water, phosphate solutions, or other buffer solutions. "Biological samples" include animal, plant, and food derived samples, including, for example, human or animal derived urine, saliva, blood and components thereof, spinal fluid, vaginal secretions, sperm, feces, sweat, secretions, tissues, organs, tumors, cultures of tissues and organs, cell cultures, and media. Preferably, the biological sample is urine, and preferably, the biological sample is saliva. Food samples include food processed materials, final products, meat, cheese, wine, milk, and drinking water. Plant samples include samples derived from any plants, plant tissues, plant cell cultures, and media. "Environmental samples" include samples derived from the environment (e.g., liquid samples from lakes or other bodies of water, sewage samples, earthen samples, groundwater, seawater, and waste liquid samples). The environmental sample may further include sewage or other waste water.

An appropriate testing element according to the present invention can be used to detect any analyte. Preferably, the test device of the present invention is used to detect small drug molecules in saliva and urine or viruses in blood samples, such as human immunodeficiency virus (HIV) and coronavirus. Of course, any samples of the above forms may be collected using a collection device or collector 200 according to the present invention, regardless of being solid or liquid at the beginning, provided that these liquids or liquid samples can be absorbed by an absorption element 207. The absorption element 207 on the sample collector 200 is mainly used to absorb the liquid sample. The absorption element herein may be made of water absorbent materials such as sponge, polyester fiber, filter paper, foam, and flocking. For example, the materials may be melamine porous material or melamine porous water absorbent material disclosed in the applicant's invention patent EP22185144.7 and can reduce adsorption of small molecules of drugs, especially for adsorption of THC. In some embodiments, the absorption element 207 can be compressed, and the compression degree is relatively greater or much greater than that of the sample application area of the testing element. The absorption element may be relatively flexible and can be compressed at the beginning. After absorbing a liquid sample, the absorption element is still relatively flexible; generally, the absorption element only needs to be placed in the oral cavity or slightly scraped in the oral cavity when collecting oral saliva, the absorption element can absorb the liquid sample, such as saliva or a saliva sample. A collection way of the collector will be described in detail later.

In some embodiments, a liquid sample absorbed by an absorption element may be a saliva sample, and the volume of a sample absorbed by the absorption element of the present invention from the oral cavity is 0.1-5 microliters.

### Downstream and upstream

Downstream or upstream is divided according to a flow direction of a liquid. Generally, a liquid flows to a downstream area from an upstream area. The downstream area receives the liquid from the upstream area, and a liquid also may flow to a downstream area along an upstream area. Here, downstream or upstream is generally divided according to a flow direction of a liquid, for example, on some materials where capillary force is utilized to promote the flow of a liquid, a liquid may overcome gravity to flow towards an opposite direction to the gravity; and in this case, downstream or upstream is divided according to a flow direction of the liquid. For example, in the test device of the present invention, when the absorption element 207 absorbs a liquid sample or a sample, the liquid sample may be compressed out of the absorption element. In some embodiments, the liquid sample compressed out contacts a sample application area 905 of a testing element. In this case, the liquid sample can flow from the upstream sample application area 905 to the downstream testing area 902, and finally to a water absorption area 901. In a circulation process, the liquid sample flows from a label area 904 to a testing area 902 on which a detection area 906 and a testing result control area 907 are provided. Antibodies or antigens are fixed onto the testing area, and there are labeled antibodies or antigens on the label area, and labels on the label area can flow with the flow of liquid. The testing area may be a polyester fiber film, and the sample application area 905 may be a glass fiber. In some embodiments, an area of the device where the absorption element 207 is accommodated is located upstream of a window area for reading a testing result, and the docking area for accommodating the handle of the sample collector is located upstream of the accommodating chamber for accommodating the sample application area. In some embodiments, the accommodating chamber includes a part of the sample application area 905 or a part of the sample application area 905 is located in the accommodating chamber. However, a part of the testing area 907, especially for the detection area 906 and the testing result control area 902 are exposed by a window area for reading a testing result or within the window area for reading a testing result.

### Gas flow or liquid flow

Gas flow or liquid flow means that liquid or gas can flow from one place to another place. In a flow process, the liquid or gas may pass through some physical structures to play a guiding role. The "passing through some physical structures" here means that liquid passes through the surface of these physical structures or their internal space and flows to another place passively or actively, where passivity is usually caused by external forces, such as flow under the capillary action. The flow here may also be a flow due to self-action (gravity or pressure) of the liquid or gas, and also may be a passive flow. Here, the flow does not mean that a liquid or a gas is necessarily present, but indicates a relationship or state between two objects under some circumstances. In case of presence of liquid, it can flow from one object to another. Here it means the state in which two objects are connected. In contrast, if there is no gas flow or liquid flow state between two objects, and liquid exists in or above one object but is unable to flow into or on another object, it is a non-flow, non-liquid or non-gas flow state.

### Detachable combination

Detachable combination means that connection relationship between two components is in several different states or positional relationship. For example, with two components being physical components, they can be separated at the beginning and then connected or combined in an appropriate first case, and separated in an appropriate second case. Physically, such separation is spatial separation without contact. Alternatively, the components are combined at the beginning, and can be physically separated from each other when appropriate. Alternatively, two objects are separated at the beginning, combined to achieve a specified function if necessary, then separated, or later combined again for a purpose. In short, combination or separation or two components or two objects can be easily made and repeated many times. Of course, the combination or separation can also be single-use. In addition, such combination can be a detachable combination between two components, or a two-by-two detachable combination between three or more components. For example, a first component, a second component, and a third component are provided, where a detachable combination is made between the first component and the second component or between the second component and the third component; and a detachable combination or separation is made between the first component and the third component. In addition, for the combination, two objects themselves can be detached or can be indirectly combined by other objects.

In the present invention, a detachable combination is made between the collector 200 and the test device 100. The collector in different states needs to be detached from the test device and combined with it after collecting the liquid sample. In some embodiments, the test device 100 is provided with a receiving area, an accommodating area or an accommodating chamber, or a docking area or a resting area. These areas are configured to allow the sample collector 200 to be combined with the test device, and in the combined body, the collector is not detached from the test device. In some embodiments, the test device includes the accommodating chamber 302, where the accommodating chamber is configured to accommodate the absorption element 207 on the collector, and the docking area 304 is used to receive the handle 201 of the collector. In some embodiments, when the collector is in different states, the state of the absorption element also changes. When the collector is detached from the test device or departs from the accommodating chamber and the docking area, the absorption element also departs from the accommodating chamber and the handle of the collector also departs from the docking area. It can be understood that the absorption element and the handle are an integrated structure. When the handle departs from the docking area, it naturally drives the absorption element to depart from the accommodating chamber and the test device (if the absorption element is located in the cover body, the cover body is also taken away together). After departing from the test device, the collector can be used to collect a liquid sample, for example, the absorption element 207 extends into the mouth to collect saliva samples, then the collector is returned to the test device again, and then the handle of the collector is returned to the docking area. When the absorption element is returned to the accommodating chamber, the absorption element is compressed to release the liquid sample, and the liquid sample naturally flows to the sample application area 905 on a part of the testing element located in the accommodating chamber, and the liquid sample flows to the downstream label area 904 under a capillary action, dissolving the label, allowing the label and the sample to flow to the downstream testing area together with the sample, and reacting with the substances fixed in the testing area and the testing result control area, thereby displaying the testing result. The area where the collector is detached from the test device is the same as the area where it enters or rests on, such that it is always detached from or enters or rests on the same area of the test device, facilitating an operation by an operator, without causing any misoperation.

### Testing element

The "testing element" used herein refers to an element that can be used to detect whether a sample or a specimen contains an interested analyte. Such testing can be based on any technical principles, such as immunology, chemistry, electricity, optics, molecular science, nucleic acids, and physics. The testing element can be a lateral flow test strip that can detect a variety of analytes. Of course, other suitable testing elements can also be used in the present invention.

Various testing elements can be combined for use in the present invention. The test strip is one form thereof. The test strips used for analyzing the analyte (such as drugs or metabolites that show physical conditions) in samples can be of various forms such as immunoassay or chemical analysis. The analysis mode of non-competition law or competition law can be applied for test strips. A test strip generally contains a water absorbent material that has a sample application area, a reagent area, and a testing area. Samples are added to the sample application area and flow to the reagent area under the capillary action. If analyte exists in the reagent area, samples will bind to the reagent. Then, samples continue to flow to the testing area. Other reagents such as molecules that specifically bind to analyte are immobilized on the testing area. These reagents react with the analyte (if any) in the sample and bind to the analyte in this area, or bind to a reagent in the reagent area. Label used to display the detection signal exists in the reagent area or the detached label area.

Typical non-competition law analysis mode: if a sample contains analyte, a signal will be generated; and if not, no signal will be generated. Competition law: if no analyte exists in the sample, a signal will be generated; and if analyte exists, no signal will be generated.

The testing element can be a test strip, which can be water absorbent material or non-water absorbent material. The test strip can contain several materials used for delivery of liquid samples. One material of the test strip can cover the other material thereof. For example, the filter paper covers the nitrocellulose membrane. One or more materials may be used in one area of the test strip, and one or more other different materials may be used in the other area thereof. The test strip can stick to a certain support or on a hard surface for improving the strength of holding the test strip.

Analyte is detected through a signal generating system. For example, one or more enzymes that specifically react with this analyte is or are used, and the above method of fixing a specific binding substance on the test strip is used to fix the combination of one or more signal generating systems in the analyte testing area of the test strip. The substance that generates a signal can be in the sample application area, the reagent area or the testing area, or on the entire test strip, and one or more materials of the test strip can be filled with this substance. The solution containing a signifier is added onto the surface of the test strip, or one or more materials of the test strip is or are immersed in a signifier-containing solution. The test strip containing the signifier solution is made dry.

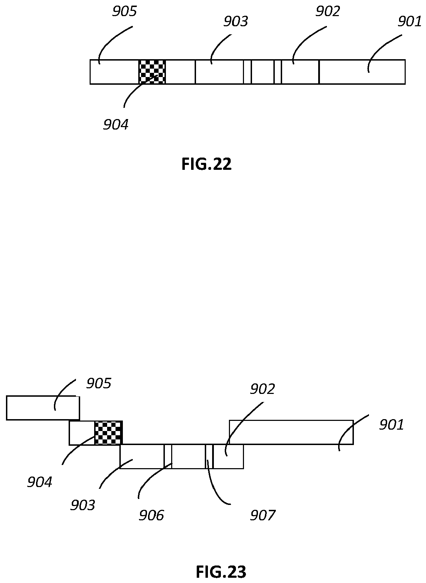

Various areas of the test strip can be arranged in the following way: sample application area 905, reagent area 904, testing area 902, control area, an area to determine whether the sample is adulterated or not, and liquid sample absorption area 901 (FIG. 22 - FIG. 24). The testing result control area is located behind the testing area. All areas can be arranged on a test strip that is only made of one material. Alternatively, different areas may be made of different materials. Each area can be in direct contact with the liquid sample, or different areas are arranged according to the flow direction of liquid sample; and a tail end of each area is connected and in overlapped with the front end of the other area. Materials used can be those with good water absorption such as filter papers, glass fibers or nitrocellulose membranes. The test strip can also be in other forms.

The nitrocellulose membrane test strip is commonly used, that is, the testing area includes a nitrocellulose membrane on which a specific binding molecule is immobilized to display the testing result; and other test strips such as cellulose acetate membrane or nylon membrane test strips can also be used. For example, test strips and similar devices with test strips disclosed in the following patents: US 4857453; US 5073484; US 5119831; US 5185127; US 5275785; US 5416000; US 5504013; US 5602040; US 5622871; US 5654162; US 5656503; US 5686315; US 5766961; US 5770460; US 5916815; US 5976895; US 6248598; US 6140136; US 6187269; US 6187598; US 6228660; US 6235241; US 6306642; US 6352862; US 6372515; US 6379620, and US 6403383. The test strips and similar device with test strips disclosed in the above patents may be applied to the testing element or test device of the present invention for the detection of an analyte, for example, the detection of an analyte in a sample.

Test strips used in the present invention may be commonly referred as lateral flow test strips. The specific structure and detection principle of the test strips are well known to a person skilled in the art in the prior art. A common test strip includes a sample collection area or a sample application area, a label area, and a testing area; the sample collection area includes a sample receiving pad, the label area includes a label pad, and a water absorption area may include a water absorbent pad, where the testing area includes necessary chemical substances for detecting the presence or absence of analyte, such as immunoreagents or enzyme chemical reagents. The nitrocellulose membrane test strip is commonly used, that is, the testing area includes a nitrocellulose membrane, and an area on which a specific binding molecule is immobilized to display the testing result; and other test strips such as cellulose acetate membrane or nylon membrane test strips can also be used. Of course, in the downstream of the testing area, there may also be a testing result control area; generally, test strips appear on the testing result control area and the testing area in the form of a horizontal line, namely, a test line or a control line. Such test strips are conventional. Of course, they can also be other types of test strips for detection under the capillary action. In addition, there are dry chemical reagent components on common test strips, for example, an immobilized antibody or other reagents. When the test strip contacts liquid, the liquid flows along the test strip under the capillary action, and the dry reagent components are dissolved in the liquid and treated in a next area, and the dry reagents react in the area for necessary detection. The liquid flow mainly relies on the capillary action. Here, all of the test strips can be applied to the test device of the present invention or can be disposed in contact with the liquid samples in a detection chamber or used to detect the presence or absence of analyte in the liquid samples that enter a detection chamber, or the quantity thereof. Generally, the testing element is configured in the detection chamber. When a liquid chamber exists in the detection chamber, it contacts the testing element for assaying or testing. The detection chamber herein includes a bottom plate 400 and a cover plate 300 between which a testing element is provided. In some embodiments, the bottom plate is provided with groove structure 403, 404, 405 for placing a water absorption area 901 of the testing element. In some embodiments, a lower housing is provided with an area 401 for accommodating an absorption element, on which the sample application area 905 of the testing element can be provided. Alternatively, the absorption element 207 can be in direct or indirect contact with the sample application area on the area 401, for example, the absorption element 207 covers the sample application area. In some embodiments, an opening is provided in the upper housing or the upper card 300, corresponding to or combined with the sample application area 401 of the lower housing (when the upper housing 100 and the lower housing 501 are combined) to form the accommodating chamber 302 for accommodating the absorption element. In this case, the sample application area 401 is provided on the lower housing to constitute the bottom of the accommodating chamber, the opening of the upper housing forms the opening of the accommodating chamber, and the depth of the opening of the upper housing is the depth of the accommodating chamber. However, the sample application area is located at the bottom of the accommodating chamber, and the absorption element can cover the sample application area when located in the accommodating chamber. The depth of the accommodating chamber herein is generally equivalent to the overall thickness of the collector with the absorption element 207, and is greater than the thickness of the absorption element 207, generally 2-8 mm or 3-5 mm.

### Analyte

Examples that can use an analyte related to the present invention include some smallmolecule substances, including drugs (such as drug of abuse). "Drug of Abuse" (DOA) refers to using a drug (playing a role of paralyzing the nerves usually) not directed to a medical purpose. Abuse of these drugs will lead to physical and mental damage, dependency, addiction and/or death. Examples of drug abuse include cocaine; amphetamine (AMP) (e.g., Black Beauty, white amphetamine tablets, dexamphetamine, dexamphetamine tablets, and Beans); methamphetamine (MET) (crank, meth, crystal and speed); barbiturate (BAR) (such as Valium, Roche Pharmaceuticals, Nutley, and New Jersey); sedatives (i.e., a sleep aid medicine); lysergic acid diethylamine (LSD); inhibitors (downers, goofballs, barbs, blue devils, yellow jackets, and methaqualone); tricyclic antidepressants (TCAs, i.e. imipramine, amitriptyline, and doxepin); dimethylenedioxymethylaniline (MDMA); phencyclidine (PCP); tetrahydrocannabinol (THC, pot, dope, hash, weed, etc.); opiates (i.e., morphine (MOP) or opium, cocaine (COC), heroin, and hydroxydihydrocodeinone); and anxiolytic drugs and sedative-hypnotic drugs. The anxiolytic drugs are mainly used for relieving anxiety, tension, and fear, and stabilizing emotion, and have hypnotic and sedative effects. The anxiolytic drugs include benzodiazepines (BZO), atypical benzodiazepines (BZ), fused dinitrogen NB23C, benzazepines, ligands of BZ receptors, open-ring BZ, diphenylmethane derivatives, piperazine carboxylates, piperidine carboxylates, quinazolinones, thiazine and thiazole derivatives, other heterocycles, imidazole-type sedative/analgesic drugs (e.g., oxycodone (OXY) and methadone (MTD)), propylene glycol derivatives-carbamates, aliphatic compounds, anthracene derivatives, and the like. The test device of the present invention may also be used for detecting drugs belonging to a medical use but easy to be taken excessively, such as tricyclic antidepressants (imipramine or analogues) and acetaminophen. These drugs are metabolized into micromolecular substances after absorbed by human body. These micromolecular substances exist in blood, urine, saliva, sweat and other body fluids or in some body fluids.

For example, the analyte detected by the pre invention includes but is not limited to creatinine, bilirubin, nitrite, (nonspecific) proteins, hormones (for example, human chorionic gonadotropin, progesterone, follicle-stimulating hormone, etc.), blood, leucocytes, sugar, heavy metals or toxins, bacterial substances (such as proteins or carbohydrates against specific bacteria, for example, *Escherichia coli* 0157:H7, *Staphylococcus, Salmonella, Fusiformis, Camyplobacter genus, L. monocytogenes, Vibrio,* or *Bacillus cereus*) and substances related with physiological features in a urine sample, such as pH and specific gravity. Chemical analysis of any other clinical urine may be performed by lateral flow detection in combination with the device of the present invention.

### Liquid flow

Typically, liquid flow refers to flow from one place to another place. Under normal circumstances, most of liquid flow in nature is from a high place to a low place under the action of gravity. Flow herein also depends on external force (namely, external gravity), and thus it can become flow under normal gravity. In addition to gravity, liquid flow can also serve gravity to move from low to high. For example, liquid can flow from a low place to a high place through extraction, compression or pressure received by it. Alternatively, liquid against its own gravity in relation to pressure can flow.

### Test device and collector

Test device refers to a device for testing presence or absence of an analyte in a sample. The test device herein can simply include a detection chamber and a testing element therein, so it can be called a test device. As explained above, the test device fits with the collector in a detachable manner.

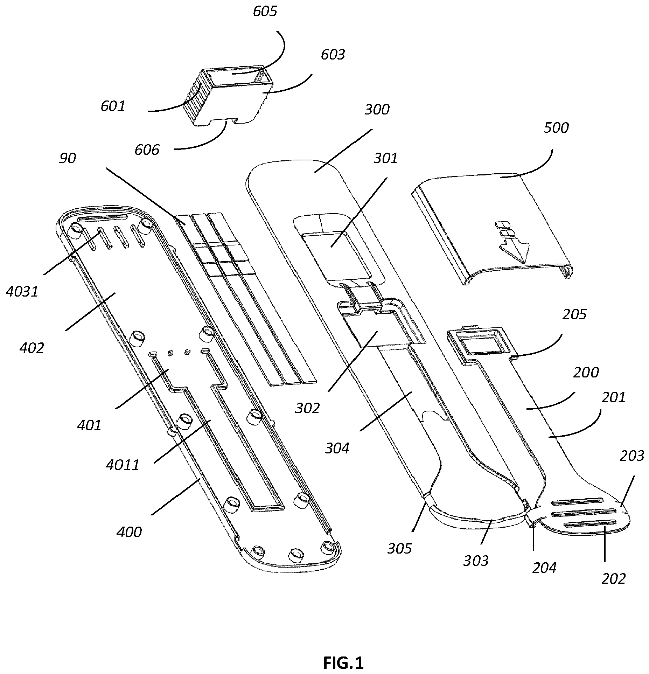

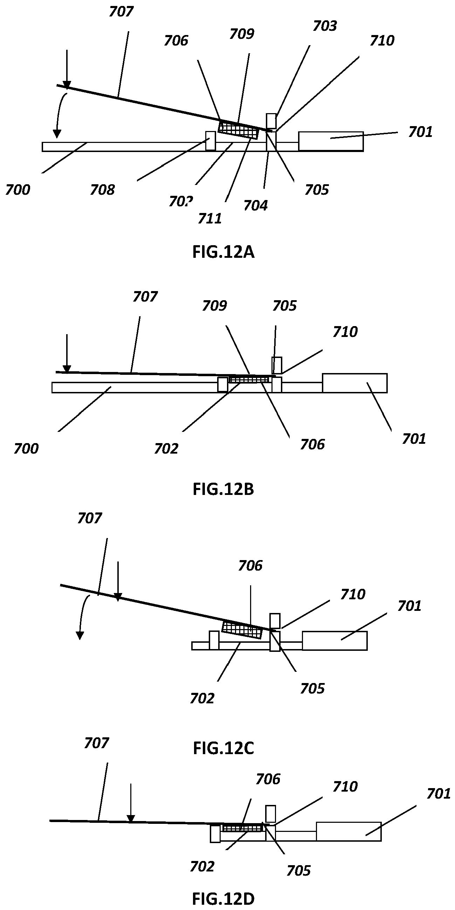

In some embodiments, for example, FIG. 1 and FIG. 12, in a specific embodiment of the present invention, the test device includes an upper housing and a lower housing, and the testing element is arranged in a specific area of the lower housing 501. For example, as shown in FIG. 1, FIG. 4, and FIG. 5, the lower housing has an area for placing the testing element, and the area includes an area 401 for placing a sample application area of the testing element 90. An area for placing a water absorption element 901 of the testing element is an area indicated by an area 4031 for placing the water absorption element 901 (FIG. 1), and the label area and the detection area are located between the area 4031 for placing the water absorption element 901 and the area 401 for placing the sample application area 905. When a plurality of test strips are placed in parallel, all sample application areas of a plurality of testing elements are located within the area 401. For example, the water absorption areas of three testing elements are arranged within areas indicated by the groove structure 403, 404, 405 of the lower housing, and are defined by raised ribs. However, the sample application areas 905 of the three testing elements are all distributed in the area 401; for example, the sample application areas may be distributed in parallel or overlapped with each other, and preferably, the sample application areas are distributed parallel to the area 401 of the lower housing. Here, the sample application areas may be partially located in the area 401 indicated or may be totally located in the area 401 indicated, the area is in a square shape as a whole, and there is also a long and narrow area 4011 in front of the area, the long and narrow area is connected with the area 401 for placing the sample application area 905, and surrounded by raised ribs 4016, 4015, 4011, 4013 for surrounding a long and square area, and the area 401 for placing the sample application area of the testing element side by side is within the square area surrounded by the raised ribs 4016, 4015. The narrow area surrounded by the raised ribs 4011, 4013 is used to support the docking area 304 on the upper card, the docking area is formed by the concave surface of the upper card, and an area 330, 302 is raised on the back of the upper card, and in contact with the raised ribs 4011, 4013 of the narrow area on the lower card, thus increasing the support strength. In order to reduce the weight of the entire test device, the upper card and the lower card are generally made of relatively light-weight materials and in the form of thin sheets, so it is necessary to form a recess in the upper card; for example, the docking area is sunken, and a protrusion 330 is naturally formed on the back thereof and located on the raised ribs 4013, 4011, increasing the overall bearing strength of the test device.

The upper card 300 of the test device includes an area 302 for accommodating the absorption element of the collector, and the area is similar to a window. When the upper card 300 is combined with the lower card 400, the accommodating chamber 302 is formed by the opening 307 of the upper card and the area 401 of the lower card for placing the sample application area of the testing element (the area 401 serves as the bottom of the chamber), such that a part or all of the sample application area 905 of the testing element is exposed through the area 302. If there are a plurality of sample application areas, the plurality of sample application areas are exposed and distributed side by side in the area, and the area 401 for placing the sample application areas constitutes the bottom of the accommodating chamber. The accommodating chamber 302 is used to accommodate the absorption element 207 of the collector. In the present invention, the accommodating chamber 302 is in a square area, and the absorption element 207 is also square, so when the absorption element 207 covers the bottom of the accommodating chamber 302 and is located in the accommodating chamber 302, the absorption element can be in contact with the sample application area of the testing element located at the bottom of the accommodating chamber 302. Of course, the absorption element 207 of the collector 200 may be of any shape, for example, circle, rectangle, ellipse, and diamond. Provided that the absorption element entering the accommodating area may be in contact with or in direct contact with the sample application area of the test strip in the accommodating chamber 302, the absorption element may also be of other shapes. If there are a plurality of testing elements in the area, the absorption element can be in simultaneous contact with various sample application areas of a plurality of test strips.

The upper housing 100 of the test device has a docking area 304, and the shape of the docking area corresponds to the shape of the handle 301 of the collector, such that the handle 201 of the collector can be rested or fixed onto the docking area. The depth of the docking area 304 for accommodating the handle of the collector is smaller than the depth of the accommodating chamber 302 for accommodating the absorption element 207; thus, when the collector needs to be removed from the test device, it is easy to directly detach the collector from the test device, especially for the upper housing 300 of the test device, or directly detach the absorption element from the accommodating chamber and depart from the test device. In order to enable the collector 200 to be stably or fixedly rested on the upper housing 300 of the test device, limiting slots 305, 306 are arranged on two sides of a tail portion of the upper housing and respectively fit with limiting cards 204, 203 on two sides of a tail portion of the collector. When the handle 201 of the collector 200 enters the docking area 304, the limiting cards 204, 203 at a tail end of the handle are respectively clamped in the limiting slots 305, 306. The limiting cards have inward elasticity, such that two sides of the elastic limiting card are clamped on two sides of the test device depending on the rebound force of the limiting card, which allows the handle 201 and a handle hand-held area 202 to be fixed in the docking area 304. The docking area 304 can accommodate the handle and the handle hand-held area 202. The handle hand-held area 202 is directly accommodated by a place indicated by the docking area 303. Generally, the absorption element is rigidly connected with the handle as an integral structure, and located in the accommodating chamber 302. However, if no handle is fixedly connected to the docking area, it is possible that the absorption element is easy to fall off from the accommodating chamber 302. Particularly, when the absorption element is in the first state, the collector is allowed to be fixed to the test device mainly depending on the fixation of the handle located in the docking area to the test device. Here, the main function of the "docking area of the handle" on the test device is to allow the handle to be fixed onto the test device or to allow the handle to be located in the groove in the form similar to the groove of the present invention or in a way that the structure of the handle and the structure of the test device are fixed together. Such fixation is detachable, it is possible to allow the handle to be fixed onto the test device in any detachable way, and the fixation range can be defined within the so-called "docking area" of the present invention.

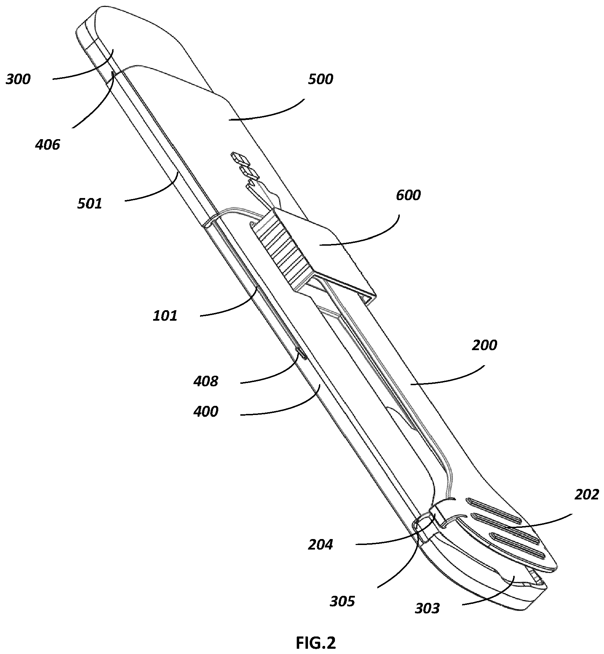

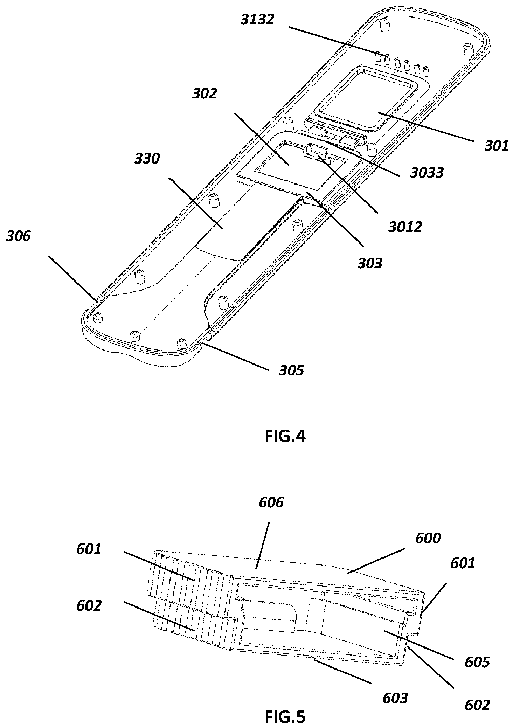

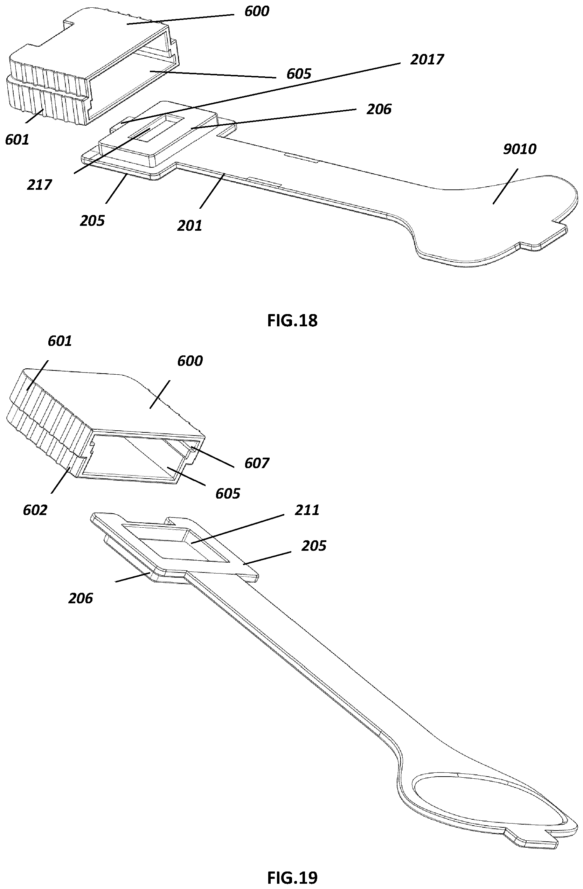

The following makes detailed explanations. The absorption element protected by the cover body 600 is located in the cover body 600 at an initial stage and the cover body is located in the accommodating chamber 302, as shown in FIG. 2. In this case, a part of the cover body is located in the accommodating chamber 302 and the part thereof is exposed. The shape of the cover body fits with that of the accommodating area, so that the cover body can be partially located in the accommodating chamber 302. Of course, it can be understood that the cover body can be located in the accommodating chamber 302 as a whole. In this case, the absorption element 207 is located in the chamber 605 of the cover body and protected by the cover body. In addition, the absorption element is also protected from being in contact with the sample application area 905 of the testing element in the accommodating chamber 302. Generally, some chemical reagents are treated in the sample application area 905 and are reagents used to improve liquid flowing properties, but there is no any label in the area. The protection of the cover body 600 is to prevent the absorption element from being in contact with the sample application area and chemical substances, such that the absorption element needs to directly extend into the oral cavity to absorb saliva during detection, and such detection is unsafe. In some embodiments, the cover body is in a step-like form. For example, as shown in FIG. 5A and FIG. 5B, the cover body has an opening; the absorption element is allowed to be inserted into the chamber 605 of the cover body through the opening, the cover body is provided with a side wall 602, 601; the circumference of the side wall 602 is smaller than that of the side wall 601, such that a part of a cover body surrounded by the side wall 602 fits with the accommodating chamber 302 and can be accommodated by the accommodating chamber, while a part of a cover body surrounded by the side wall 601 is exposed out of the upper surface of the upper card 300 of the test device, instead of being accommodated by the accommodating chamber 302. In this way, the cover body 600 is substantially located in the accommodating chamber freely, and the cover body itself has no structural fixation with the accommodating chamber, so the cover body is easy to fall off from the accommodating chamber 302. In order to prevent the cover body from being easily detached from the accommodating chamber 302, the handle indirectly connected with the absorption element 207 is fixed onto the docking area 304 and the cover body with the absorption element may be directly fixed in the accommodating chamber 302; in this case, the absorption element is in the first state and protected by the cover body, without absorbing the liquid sample; and in the first state, the absorption element is not in direct contact with the sample application area of the testing element. In order to prevent the cover body from being in direct contact with the sample application area, a ring-shaped step 308 is provided in the accommodating chamber 302, and surrounds the inside thereof and extends out of the accommodating chamber 302, such that the accommodating chamber 302 is divided into two different parts, one is an area of an upper part of the step (an upper part of the accommodating chamber), the upper part of the step 308 and an opening of the accommodating chamber form a large area part, while a lower part of the step 308 and the area 401 of the lower housing501 for placing the sample application area form a lower part of the accommodating chamber (the lower part of the accommodating chamber). It can be understood that the accommodating chamber 302 takes the area 401 of the lower card as a bottom thereof and has an opening 307, and the depth of the accommodating chamber 302 depends on the depth of the area 401 of the lower card and the depth of the opening of the upper card. In this way, when the cover body 600 is placed in the accommodating chamber 302, a lower surface 603 of a small part of the cover body is in contact with a surface of the step 308, such that the cover body is not in direct contact with the sample application area of the testing element at the lower part of the accommodating chamber 302 at the bottom 401 thereof. The sample application area is generally made of water-absorbent material, for example, fiberglass, for receiving a liquid sample. A part of the cover body protrudes from the accommodating chamber; and when a movable application element is located in the window area, the sliding element is limited to slide because of the obstruction of the cover body (as shown in FIG. 2). Generally, the absorption element 207 is loaded by a rigid carrier 206. For example, the absorption element 207 is bonded to a surface of the carrier 206, and the handle of the collector is rigidly connected with the carrier, for example, one-time injection molding. Then, the rigid carrier is also located in the cover body 600 and protected by the cover body. In this way, the accommodating chamber 302 does not actually affect the cover body therein. In order to allow the collector to be stably located on the test device, the handle 201 of the collector 200 is located and fixed in the docking area, such that the collector is fixed onto the test device as a whole. When the collector fits with the cover body 600, two edges 2018 of a frame body 205 of the collector are inserted into the cover body through the groove 607 of the cover body, and the absorption element is located in the chamber of the cover body (as shown in FIG. 19 and FIG. 18).

In actual design, the back face 313 of the ring-shaped step 308 is located at the raised ribs 4016, 4015 of the lower card 400 around the sample application area. Generally, the thickness of the sample application area is smaller than the height of the raised ribs 4015, 4016; thus, when pins 3055, 3056 (FIG. 4) on the upper card are inserted into the jacks 4022, 4021 on the lower card, the accommodating chamber 302 is allowed to form two chambers with different sizes, the chamber below the step 308 has a small opening, and the chamber above the step 308 has a large opening, such that the cover body can be located in the accommodating chamber 302, but above the step 308 without being in contact with the sample application area of the test strip, and a part of the cover body is located outside the accommodating chamber 302. Thus, the absorption element with the cover body 600 can be fixed on the test device. When the absorption element is detached from the cover body and enters the accommodating chamber 302 again, the absorption element can be allowed to be located below the step 308, thus covering the sample application area below the step 308. In such a design, it is not desired that when the absorption element is located on the cover body, the cover body is in contact with the sample application area; it is desired that when the absorption element is detached from the cover body, the absorption element is in contact with the sample application area during the detection; in this case, the absorption element can be totally located in an area below the step 308 of the accommodating chamber 302, and can totally cover the sample application area. In addition, the sample application area is limited by the raised ribs 4016, 4015 around the bottom of the accommodating chamber 302. Excess liquid on the absorption element, if any, will be distributed in the area 401 without flowing to other parts of the lower card, such that more liquid can flow to the sample application area to be tested, thereby ensuring that the liquid on the absorption element is sufficient for the test strip to flow and flows to the absorption area to complete the test.

### Collector and accommodating chamber

The collector of the present invention is different from a typical collector. When the collector of the present invention is in an initial start state, the absorption element of the collector is protected by the cover body and located on the test device; when the absorption element is required to be tested, the collector is detached from the test device to collect a sample and then returned to the test device again; and when the collector is returned to the test device, the absorption element of the collector is compressed to release the liquid.

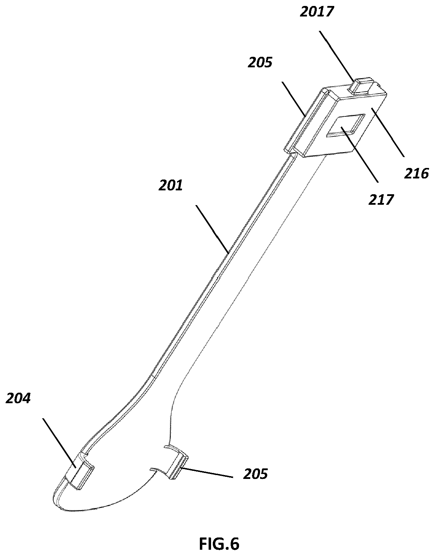

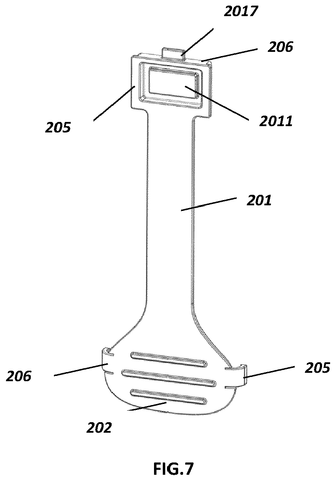

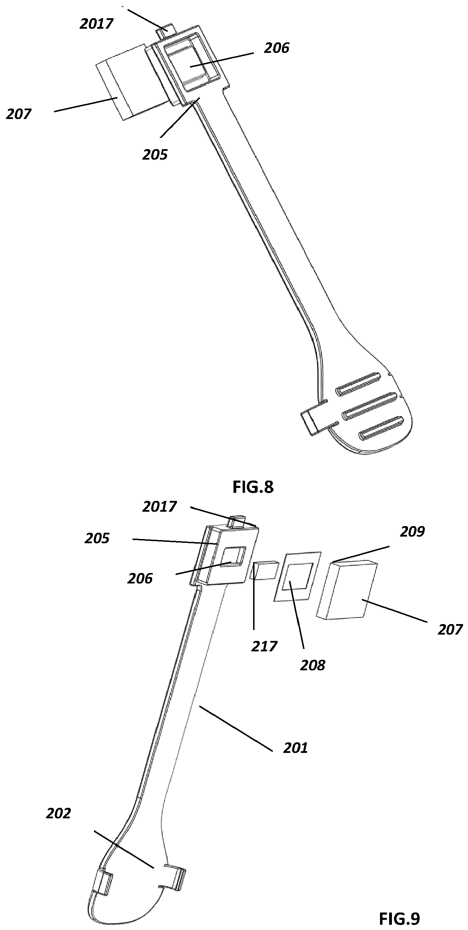

For example, as shown in FIG. 6 to FIG.9, the collector 200 includes a handle 201 and a carrier 206 connected to the handle; the absorption element 207 is loaded on the carrier and mainly used for absorbing the liquid sample; in addition, pressure is applied to the absorption element to be tested, such that the absorption element is compressed, thereby releasing the liquid sample from the absorption element. The carrier of the collector is of a rigid plastic structure. In some embodiments, the carrier and the handle are connected into an integral structure, and the carrier is loaded with the absorption element 207 to form an absorption head of the collector, and the absorption head has a thickness, and the thickness is equivalent to or substantially the same as a depth of the accommodating chamber. In order to form such thickness, it is possible to allow a thickness of the carrier to be directly equivalent to the depth of the accommodating chamber 302; thus, the absorption element can be in direct contact with the sample application area at the bottom of the accommodating chamber when entering the accommodating chamber. In some embodiments, the carrier is made of transparent material and is of a thin plate structure, and the carrier is embedded on a frame body 205 of an opening 211. A recess 218 is formed in the frame body, and a back face 216 of a thin plate can be seen through the recess 218. The thin plate as a surface covers the frame body, such that the front face 215 of the thick plate is loaded on the absorption element 207. A loading way can be that the absorption element is bonded to the front face 215 of the thin plate 206 through an adhesive layer 209. In some embodiments, there is a recess 217 in the thin plate 206, and an indicator element 208 for indicating whether a liquid sample is sufficient is placed in the recess 217. The indicator element is located in the recess 217 of the thin plate 206; thus, when the absorption element covers a label of the carrier to protect the thin plate 206, the indicator element covers the absorption element 207. Because the thin plate is transparent, a change in color of the indicator element 208 on the thin plate 206 can be observed through the recess 218. When the absorption element 207 absorbs sufficient liquid, the liquid will moisten the indicator element 208, and the indicator element will change color thereof, indicating that the absorption element has absorbed a sufficient amount of liquid samples. It can be observed that the thin plate is transparent through the back face 216 of the sunken thin plate. The indicator element 208 covers the absorption element 207, and the volume of liquid absorbed by the absorption element is very small, ranging from 1 microliter to 100 microliters, with a maximum of 500 microliters. Therefore, the indicator element 208 is just a sheet and absorbs a small amount of liquid enough to be moistened, thus displaying color, indicating that the absorption element has collected sufficient liquid. The structural layout of such indicator strips is different from that of a typical collector. The absorption element of a conventional collector is connected to one end of a long indicator strip. During the collection of liquid, liquid on the absorption element will flow to the indicator strip and display color. However, the present invention only requires a very short piece, such that the entire indicator strip can cover the surface of the absorption element and develop color faster. Display color herein may be white or colorless in a dry condition, red or other colors in a wet condition, for example, blue and pink. As shown in FIG. 9 and FIG. 8, the carrier with the absorption element is of a flat structure, and the shape of the carrier fits with that of the accommodating chamber 302, such that the carrier 206 can be allowed to enter completely into the accommodating chamber 302, and the absorption element 207 is allowed to face downward and can be in direct contact with the sample application area 905 at the bottom of the accommodating chamber.

After the absorption element of the collector is allowed to absorb the liquid sample, the collector is returned to the test device again, allowing the carrier 206 with the absorption element 207 to directly enter the accommodating chamber302 along with the absorption element being compressed, thereby releasing the liquid sample to the sample application area of the testing element. In other words, when the carrier 206 is allowed to enter the accommodating chamber 302, a pressure is applied to the absorption element, thereby compressing the absorption element and automatically releasing liquid onto the sample application area. Therefore, in the present invention, an insertion element 2017 is arranged at a top of the collector with the absorption element 207 and extends forwards on a front frame of a frame body 205. The insertion element is similar to a protruding tongue and is rigid. The frame body 205, the thin plate 206, and the insertion element 2017 may be injection molded in one time. Of course, the handle 201 connected to the frame body 205, the handle hand-held area 202, and the elastic limiting cards 204, 203 distributed on two sides of the handle can be simultaneously injection molded in one time. The insertion element 2017 is provided on the collector, and a jack is naturally provided in the accommodating chamber 302 to allow the insertion element to be inserted therein.

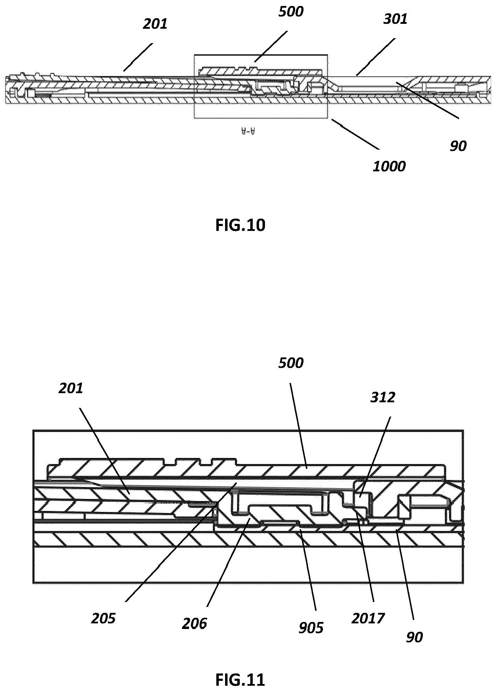

Therefore, the accommodating chamber 302 includes a jack 312 in which the collector is allowed to be inserted, and the insertion element 2017 on the collector can be inserted into the jack 312. In a specific embodiment, the insertion element 2017 of the collector is provided at an intermediate position of the carrier of the collector, and the jack in the accommodating chamber is also provided at an intermediate position of the accommodating chamber and proximal to the window area for reading a testing result. Specifically, the jack 312 is a small chamber raised on an annular edge of the accommodating chamber, and the small chamber has a specific depth and divides a part of the annular edge into two parts 310, 3311; the jack or the small chamber has a specific depth, which can allow the insertion element 2017 of the collector to be inserted into the jack 312. In some embodiments, the small chamber or the jack has an upper wall 309; as a part of an upper housing, the upper wall 309 serves as a partition between the accommodating chamber 302 and the window area 301 for reading a testing result. The cover body being located in the accommodating chamber is provided with a notch 606, allowing the upper wall 309 to be located in the notch, which can limit left and right positions of the cover body. In order to allow the collector 200, especially for the carrier 206 with the absorption element 207, to enter the accommodating chamber 302, the absorption element 207 can be extruded and release liquid, and the liquid flows to the sample application area. Now, referring to FIG. 11A to FIG. 11D, theoretical descriptions are made, for example, how the collector causes the absorption element 207 to be compressed and release a liquid sample when inserted into the accommodating chamber 302.

For example, in FIG. 12A to FIG. 12D, a collector with a handle 707 is provided; a carrier 709 is provided on one end of the handle and provided with the absorption element 706 passing through the test device; the test device includes the accommodating chamber 702 for accommodating the carrier with the absorption element; the chamber is surrounded by side walls 708, 704; a sample application area of a testing element is arranged inside the bottom of the accommodating chamber 702; and the testing area is located downstream 701 of the accommodating chamber. The jack 710 is formed on the side wall 704 and formed by a side wall at one end and an upper structure 703, and an insertion element 705 is provided at a tail end of the collector. During operation, for example, the absorption element 706 of the collector absorbs a liquid sample. Because the accommodating chamber 702 is sunken, a common operation is that the entire collector is allowed to be inserted into the accommodating chamber 702 at an inclined angle (FIG. 12A). In particular, the insertion element 705 of the collector is allowed to be obliquely inserted into the jack 710. After insertion, a front end portion of the absorption element may be partially in contact with the sample application area (similar to a pivot 711), as shown in FIG. 12A. When the handle 707 obliquely inserted continues to be operated, it moves in a direction proximal to the test device and remains parallel to the test device 700 or is fixed onto the test device 700; for example, the handle 707 is fixed into the docking area of the test device. When the handle 707 of the collector moves, the position of the pivot 711 does not change, and the insertion element 705 in the jack 710 is fixed but can move up and down. The insertion element 705 may be in contact with the upper structure 703 of the jack 710, and the carrier 709 also drives the absorption element to move to the bottom of the accommodating chamber with the downward movement of the handle 707, finally covers the accommodating chamber 702, and gradually covers the sample application area in the accommodating area. During the movement of the handle, the handle indirectly applies a downward pressure to the carrier 709, and the pressure is transmitted to the absorption element through the carrier. The sample application area in the accommodating chamber 302 is located on the rigid bottom plate of the lower housing, such that the absorption element 706between the sample application area and the rigid carrier 709 may be easily compressed, thereby releasing the liquid sample. The test device of the present invention is operated specifically in the above manner; thus, when the absorption element on the collector absorbs liquid, the absorption element is returned to the accommodating chamber, and the handle is returned to the docking area again, during which the absorption element is compressed to release the liquid sample.

In another embodiment, as shown in FIG. 12C and FIG. 12D, the test device has no an area for accommodating the handle 707 of the collector, and the handle is suspended. However, the handle moves in a same way as described above, such a way is to reduce the length of the test device and only retain the accommodating chamber including the sample application area and the window area for reading a testing result, thereby shortening the length of the test device. This is mainly because the test device is generally made of plastic materials and the recycling cost of the plastic materials and the environmental protection requirements thereof are getting higher and higher. If the length of the test device is shortened, the plastic materials will be naturally reduced, the recycling cost thereof and the cost of environmental protection will also be reduced. Other operation procedures are the same as those described in FIG. 12A and FIG. 12B. In this case, although the handle is not fixed onto the test device, the test device still has the function of applying pressure to the carrier 709 through the handle and the same effect that the absorption element 706 can be compressed by squeezing to release liquid.

Therefore, it can be learned from the above description that the collector is combined or fixed together with the test device before detection, and located at a specific position thereon; when departing from the position, the collector collects a liquid sample and then is returned to the above position of the test device; although the positions on the test device are the same, a connection relationship or specific combination structure between the collector and the test device is different. For example, as shown in FIG. 2, this is a schematic diagram of a combination of the collector and the test device and the absorption element being in the first state. The collector is provided with the cover body 600 located in the accommodating chamber 302, and the cover body includes the carrier 206 with the absorption element 207 and the insertion element 2017. The handle of the collector is located in the docking area 304 of the test device, that is, the handle of the collector is located at initial positions of the collector and the test device, which is one of specific combination forms of the collector and the test device. When the absorption element is required to be tested, the cover body 600 is removed from the collector 200 to expose the carrier 206 with the absorption element 207 and the insertion element 2017 thereon; after the absorption element 207 absorbs the sample, the collector is returned to a same position; in this case, the collector has no the cover body 600, but passes through the insertion element 2017 and is inserted into the jack 312 in the accommodating chamber 302 to be fixed; the carrier with the absorption element 207 is returned into the accommodating chamber and is bonded and fixed to the accommodating chamber; the handle 201 is still located in a same docking area 304; and a combination form and position of the handle and the test device does not change. The "position" on the test device can be referred to as an area for accommodating and fixing the collector, and the area includes the accommodating chamber for accommodating the carrier 206 with the absorption element 207, the handle 201 for accommodating the collector, and the docking area 304 of the handle hand-held area 202. The so-called "accommodating" mentioned in accommodating the carrier with the absorption element 207 includes indirect accommodating and direct accommodating; the indirect accommodating means that the cover body 600 is accommodated to accommodate the carrier 206 with the absorption element 207 (which is located in the cover body); and the direct accommodating means that the carrier with the absorption element 207 directly enters the accommodating area and is fixed together with the accommodating chamber 302. At the position, the collector has a combination relationship different from the test device, which can also be expressed as the fact that the collector exists in a different state on the test device. It can be understood that, in a specific embodiment of the present invention, when the absorption element is in the first state and the second state, the handle of the collector is located at a same position of the test device, the handle and the test device share a specific fixing structure and way, and the absorption element and the accommodating chamber share a positional relationship but have a difference in a specific interaction structure; in the first state, the absorption element is protected by the cover body and is not directly located in the accommodating chamber; however, in the second state, the absorption element directly covers the accommodating chamber, and the insertion structure is also allowed to be inserted into the accommodating structure of the accommodating chamber. In a specific embodiment of the present invention, the insertion element of the absorption element is inserted into the jack of the accommodating chamber, which is one of ways of interaction between the collector and the accommodating chamber but not the only way thereof. For example, the collector has no insertion element and the accommodating chamber has no jack, but the sample end of the collector has the carrier. The shape and size of the carrier are equivalent to the internal shape and size of the accommodating chamber, such that the carrier can directly enter the accommodating chamber by applying pressure to the back face of the carrier and is embedded into the accommodating chamber; when the carrier and the accommodating chamber are made of plastic materials, the carrier is allowed to be located in the accommodating chamber through an interaction force; the pressing force of the carrier causes the carrier to be located in the accommodating chamber, and at the same time, pressure is applied to the absorption element such that the absorption element is compressed to release liquid. In this case, the handle of the collector does not need to play a role, but the way is actually an additional step from the specific operation and a little cumbersome, but it is also a feasible way.

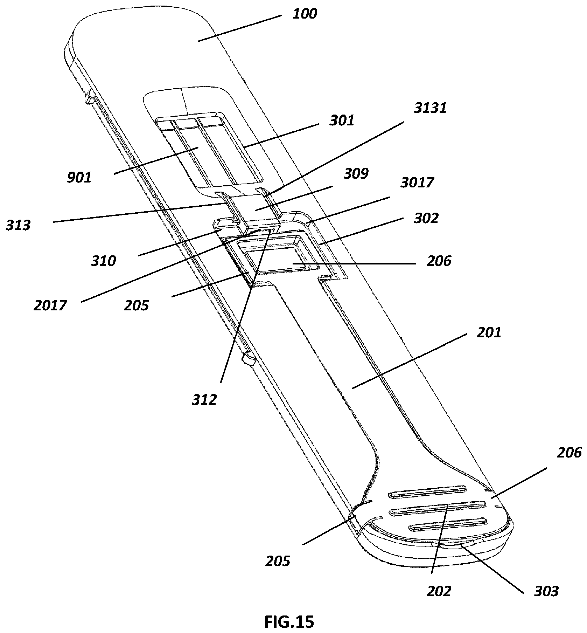

When the collector that has collected the liquid sample is combined with the test device again, the collector is combined with the accommodating chamber 302 using a process illustrated in FIG. 12A and FIG. 12B, such that the absorption element 207 can be compressed to release the liquid sample. For example, as shown in FIG. 15, FIG. 15 is a schematic diagram of a structure showing that the collector has been located in the docking area after absorbing the liquid sample, and the absorption element has been located in the accommodating chamber 302; however, before the collector enters the test device using a process illustrated in FIG. 12A and FIG. 12B, the absorption element can also be allowed to be compressed to release liquid. However, in order to allow the absorption element to be more stably located in a compression device, the absorption element is continuously compressed or given a greater pressure, such that a liquid sample on the absorption element can be released as much as possible, and the carrier 206 is given a greater pressure or a continuous pressure. Therefore, in some embodiments, the test device further includes a pressure element, the pressure element can apply the greater pressure or the continuous pressure to the carrier, such that the absorption element is subjected to a greater extrusion force to more thoroughly release liquid, or given the continuous pressure to position the absorption element in a stable pressure.

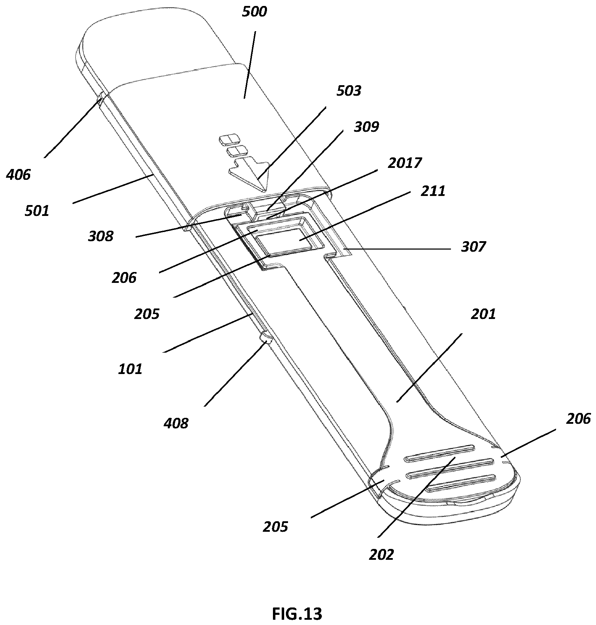

In some embodiments, the pressure element can move or slide on the test device, and preferably, slides along a general axis of the test device. In some embodiments, the pressure element has a first initial position and a second initial position on the test device. In the initial positions, the pressure element 500 is located in the window area 301 for reading a testing result and covers the window area, thereby covering the testing area of the testing element in the window area (as shown in FIG. 12). The pressure element covering the window area 301 for reading a testing result in the initial positions can protect the testing area of the testing element in the window area from being damaged, and expose only the area for accommodating the collector, and in particular protect the accommodating chamber 302 for accommodating the carrier 206 with the absorption element 207. Thus, when the collector needs to be combined with the test device again and because the window area is protected by the pressure element, the collector 200, especially for the absorption element 207 on the collector, can only unconditionally enter into the accommodating chamber 302. On the contrary, if the window area 301 for reading a testing result is not covered by the pressure element 500 but exposed, if the test device is used as a home selftesting product or an OCT product, and when the collector departs from the test device, collects a liquid sample, and is returned to the test device, an operator may insert the carrier 206 with the absorption element 207 into the window area at the beginning due to the fact that the window area 301 for reading a testing result is relatively proximal to the area 302 for accommodating the carrier 206 with the absorption element 207 (as shown in FIG. 13). This will damage the testing area within the window area. Especially when a nitrocellulose membrane is within the testing area, and if the nitrocellulose membrane is damaged, for example, being scratched, this will directly affect the results of subsequent test; in addition, if the testing area is directly exposed, the absorption element may be in contact with the testing area during the insertion of the collector, and the liquid on the absorption element may moisten the testing area, which may also directly affect the accuracy of the testing results. If it is found that the collector being inserted in a wrong position is inserted at a correct position again, this gives uncertainty to the operator and is prone to cause unfriendly operation, thereby affecting the acceptability of products. Thus, the pressure element covers the window area 301, giving the operator the certainty of the position where the collector is allowed to be inserted into the test device, which can guide the operator to perform a more accurate operation. When the absorption element 207 of the collector is provided within the accommodating chamber 302, the pressure element 500 is allowed to slide from a position covering the window area 301 onto the accommodating chamber 302 for accommodating the absorption element, and the pressure element 500 can continuously apply pressure to the absorption element or increase the pressure thereon (as shown in FIG. 13).

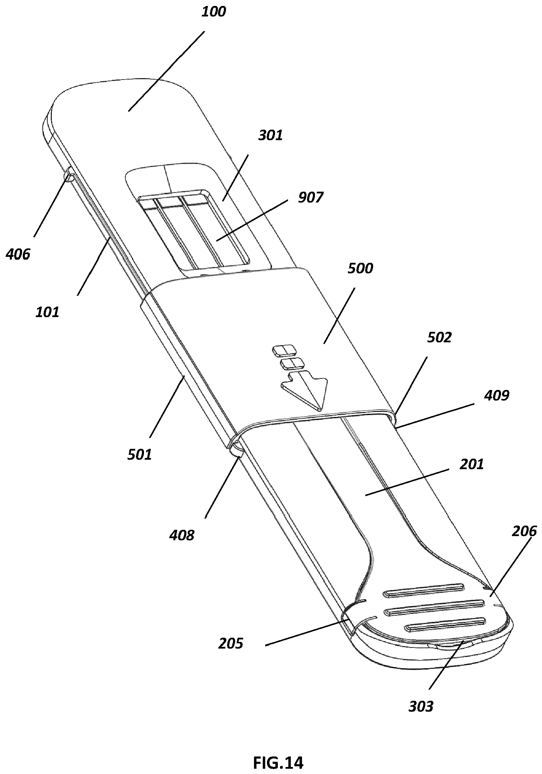

In order to allow the pressure element 500 to slide on the test device, a sliding rail or a chute 313, 3131 is provided on the pressure element, and a sliding rail or a chute is provided on the test device, which enables the pressure element to slide on the test device. In some embodiments, the pressure element has clamping parts 501, 502 that are respectively clamped on two sides of the test device, and the sliding rail 101 is respectively arranged on two longitudinal sides of the test device, and the chute (omitted) is arranged in the clamping parts, such that the chute on the pressure element can slide along the sliding rails. The cooperation between the chute in the clamping part and the sliding rail 101 on the test device not only allows the pressure element 500 to slide on the test device, but also prevents the pressure element 500 from departing from the test element. When the pressure element slides onto the accommodating chamber, a pressure is applied to the carrier 206 or the frame body 205 with the carrier and applied to the absorption element 206, and the pressure element actually receives a counter-acting force from the frame body 205. The counter-acting force substantially does not cause the pressure element to depart from the test device or the pressure element substantially does not have any specific departure change with respect to the test device, which is limited by the chutes on the clamping parts of the sliding rail 101 and the pressure element 500. Generally, the heights of the frame body 205 of the collector, the carrier 206 and the absorption element are slightly higher than the depth of the accommodating chamber 302. In this case, the pressing element slides onto the frame body 205 to give the frame body a pressing force. As shown in FIG. 13, with the collector with the absorption element 207 entering the position on the test device again, generally, if the position where the collector is inserted into the accommodating chamber 302 has any deviation, or if the handle of the collector being fixed in the docking area of the test device has a height deviation or error, the carrier 206 and the frame body 205 with the carrier 206 will be slightly higher than the accommodating chamber 302. In this case, the pressure element 500 slides from the window area 301 for reading a testing result to the accommodating chamber 302, applying a pressure to the frame body 205. The pressure is transmitted to the absorption element 207, such that the absorption element is in a compressed state continuously, the frame body 205 sinks into the accommodating chamber 302, and the absorption body 207 is compressed by great force, thereby releasing more liquid. Because the absorption element has the compressibility, a great extrusion force or continuous pressure is given to the absorption element when the pressure element 500 slides into the accommodating chamber 302, such that the liquid on the absorption element can be continuously or completely released. In order to allow the pressure element to more accurately apply pressure to the carrier with the absorption element 207 on the collector, the chutes 313, 3131 are arranged on two sides of the jack of the accommodating chamber, and the sliding rails are correspondingly arranged on the pressure element, such that when the pressure element 500 slides from the window area 301 to the accommodating chamber 302 (accommodating chamber), and the chutes on two sides of the jack 312 limit left and right positions of the pressure element 500, allowing the pressure element 500 to be accurately located on the accommodating chamber 302 and applying the pressure to the absorption element 207 (FIG. 14). In some embodiments, the pressure element is further provided with the sliding rails that respectively fit with the chutes 313, 3131 on two sides of the protrusion 309, such that the pressure element can keep a sliding direction determined in a sliding process. It can be seen from the cross-sectional diagram of FIG. 10 and FIG. 11 that when the pressure element covers the accommodating chamber 302, the pressure element acts directly on the frame body 205, the frame body is provided with the carrier 206, and the absorption element 207 is located on the carrier 206 and naturally compressed against the sample application area 905.

In some embodiments, in order to limit the sliding distance of the pressure element, limit structures 406, 408 are provided on the test device and limit the initial position of the pressure element and the sliding distance, which is equivalent to the pressure element sliding between the limit structures 406, 408. Of course, it can be understood that the slidable pressure element is a preferred embodiment of the present invention, but not a necessary condition.

In another embodiment, the pressure element is of a clamshell type. One end of the pressure element is hinged on the device, for example, it is hinged on the top surface of the top card between the window area 301 and the accommodating chamber 302 on the test device (the specific structure is omitted). In the initial state, when the absorption element on the collector is still located in the accommodating chamber of the test device, for example, when the handle of the collector is located in the docking area of the test device, the carrier with the absorption element is located in the cover body 600, and the cover body (further including the cover body 600) is located in the accommodating chamber 302, the pressure element covers the window area for reading a testing result (the first position or the first state). When the collector departs from the test device, and after the cover body is removed, the absorption element absorbs the liquid sample, and the collector is returned to the test device according to the methods described in FIG. 12A and FIG. 12B, the absorption element is allowed to be compressed under the action of the collector and the accommodating chamber. Then, the pressure element is turned over and buckled on the accommodating chamber (the second position or the second state), and pressure is applied to the frame body 205 on the collector. In order to allow the pressure element to provide a relatively large pressure, the pressure element is allowed to be totally sunken into the accommodating chamber 302. Depending on an interaction force between the pressure element and the edge inside the inner wall of the accommodating chamber 302, common plastic parts have an elastic strain that allows the pressure element to be firmly sunken into the accommodating chamber 302, overcoming the counter-acting force of the frame body 205, thereby allowing the absorption element to be continuously extruded to release liquid to the sample application area of the accommodating chamber 302.

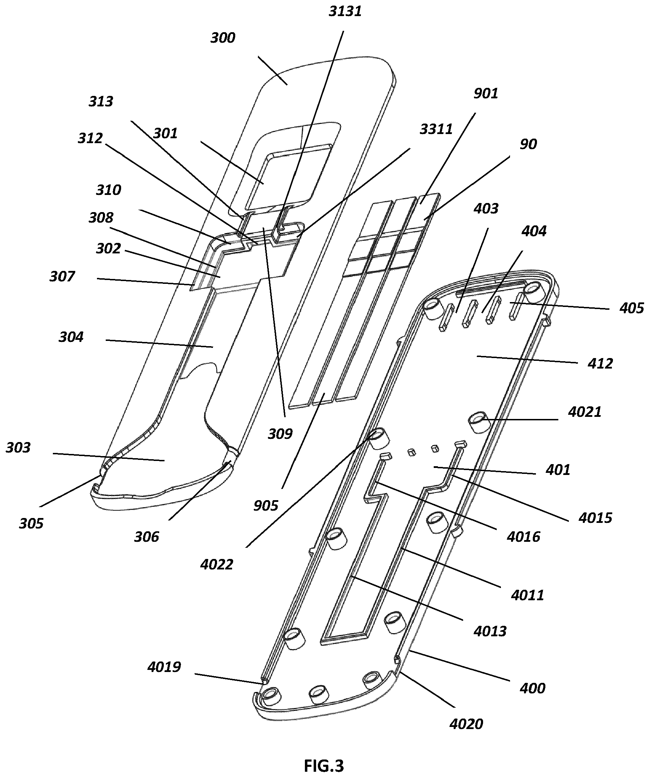

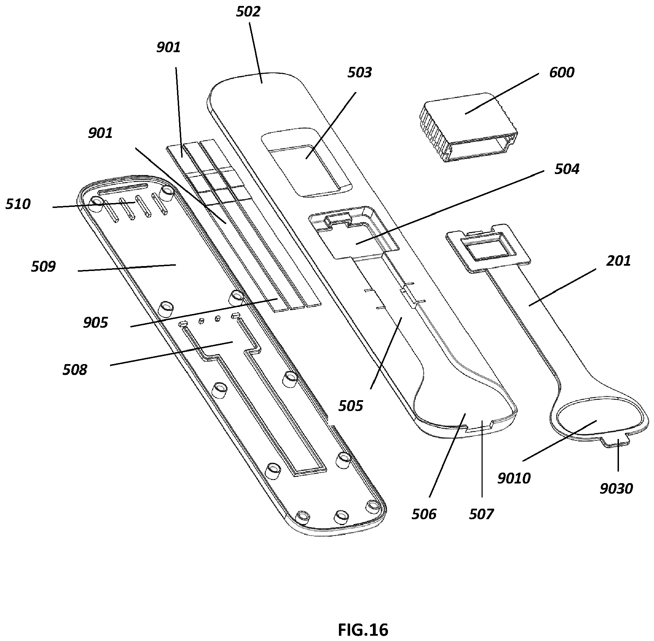

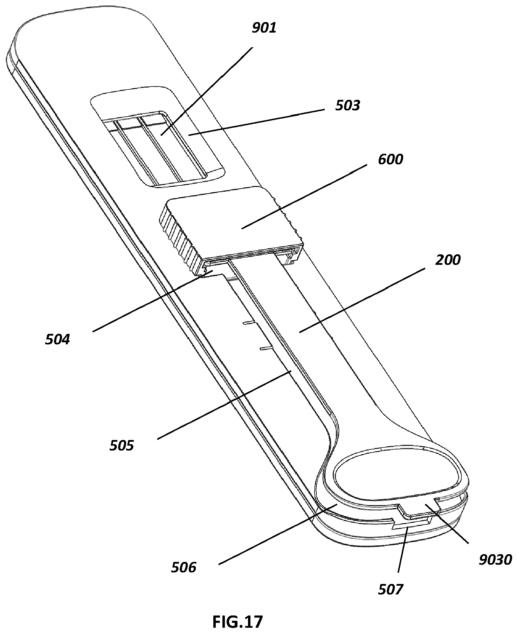

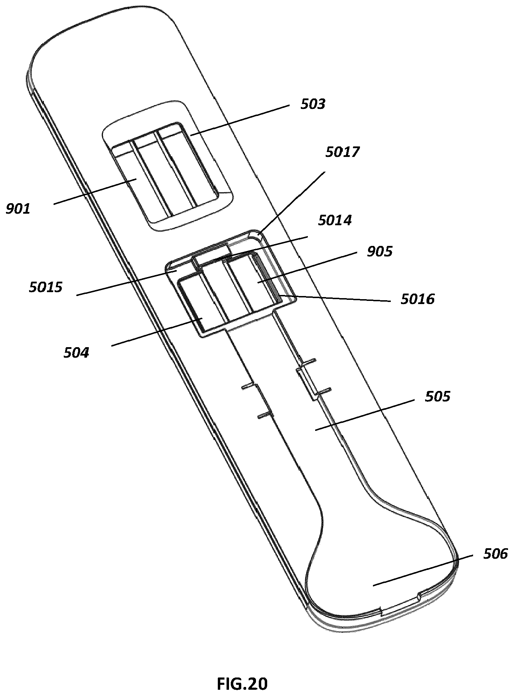

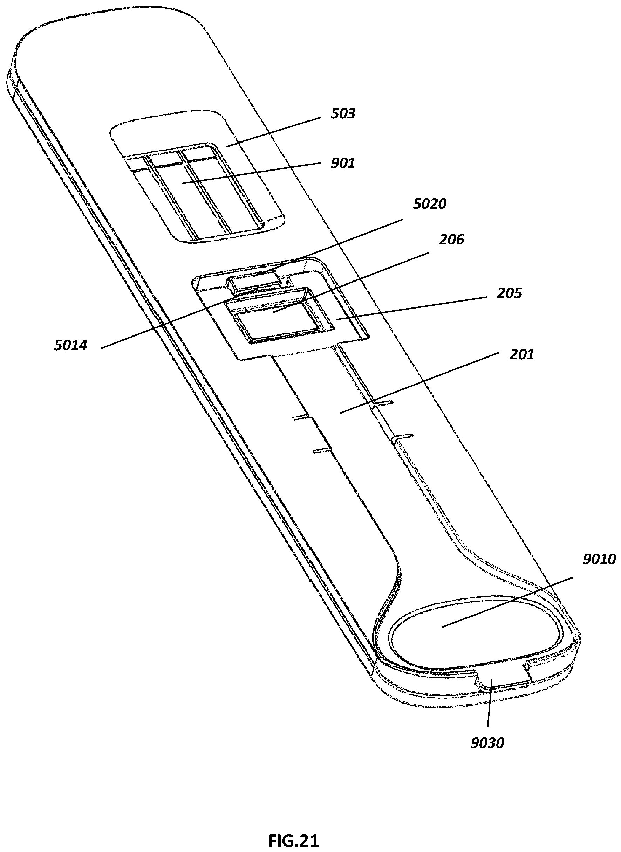

The present invention provides another specific embodiment, for example, the test device of the present invention shown in FIG. 16 to FIG. 23. The test device includes an upper housing 502 and a lower housing 501, the plurality of test strips 90 are located between the upper housing and the lower housing. Specifically, the lower housing 501 is provided with areas for placing the test strips; in such areas, the area 508 is an area for placing a plurality of sample application areas; the water absorption area for placing the test strips is located at the top 510 of the housing; the area between the top 510 and the sample application area is the testing area of the test strip and the label area thereof. Generally, the testing area is corresponding to the window area 503 for reading a testing result of the upper housing, and the sample application area is corresponding to an opening of the accommodating chamber of the upper housing. The accommodating chamber 504 of the upper housing is connected with a docking area 506 for accommodating the handle of the collector and a bayonet 507 for fixing the handle, such that a protruding structure 903 at a tail end of the handle is clamped in the bayonet to play a fixing role. Similarly, in the initial state, the absorption element 207 of the collector 200 and the carrier 206 with the absorption element are both located in the cover body 600, while the cover body is located in the accommodating chamber 504. In this case, the handle of the collector being fixed into the docking area 505 causes the cover body to be fixed into the accommodating chamber or a part of the cover body to be located in the accommodating chamber 504, and the absorption element may be in the initial state or may be in a delivery state where the product is together assembled (as shown in FIG. 17), instead of being tested. FIG. 18 and FIG. 19 show a combination of the cover body and the collector. Similarly, the cover body includes the chamber 605 of the cover body, and the collector has the handle 201 and the frame body 205 integrally formed with the handle. The carrier 206 is arranged on the frame body, and the absorption element is loaded on a front surface of the carrier. A groove 217 is provided in the carrier, an indicator element is arranged in the groove and totally covers the absorption element, and the carrier is transparent. An end portion of the collector extends out of the insertion element 2017. These structures may be located in the cover body, while the handle is exposed and may be located in the docking area of the test device. The frame body has a thickness and a width of the frame body is greater than that of the carrier, such that an edge 2018 is left around the carrier and can slide into the cover body through the sliding rail 607. Similar to FIG. 3, the entire test device has the window area 503 for reading a testing result, the window area 503 exposes the testing area of the testing element, and the accommodating chamber 504 exposes the sample application area 905 of the testing element. An annular edge 5089 is still provided in the accommodating chamber, by which the accommodating chamber is divided into an upper part and a lower part; the lower part is composed of an opening 5016 and an area 508 of the lower card; and the upper part is composed of the annular edge and an opening of the accommodating chamber. A jack 5014 is provided in the annular edge proximal to the window area for reading a testing result. The jack is absent from the annular edge, but has a protrusion 5019. The protrusion 5019 divides the annular edge into two parts 5015, 5017. The docking area 505 connected with the accommodating chamber is used to accommodate the handle 201 of the collector. In addition, a notch 507 is provided in the docking area 505 and clamped together with the protruding structure 903 at the tail end of the collector. The docking area 505 also has an area 506 for accommodating the handle hand-held area 9010 (FIG. 20).

When the absorption element is required to be tested, the collector is removed from the test device, and the cover body 600 and the collector together depart from the test device; in this case, the test device has no collector, and the accommodating chamber 504, the docking area 505, and the handle hand-held area 506 have no handle, as shown in FIG. 20. The cover body 600 is removed to expose the absorption element and the carrier 206 loaded thereon. Then, the handle hand-held area 9010 of the collector is held by hands. The absorption element is extended into the mouth or the oral cavity, such that the absorption element absorbs a saliva sample in the oral cavity for about 2-3 seconds, the saliva sample is taken out to observe whether there is red color on the carrier (the indicator element has changed color), and timely observe whether there is color in the recess 215 of the frame body 205 through the back face of the transparent carrier 206; if there is color in the recess, this indicates that the absorption element has absorbed a sufficient saliva sample. Then, the collector is allowed to be returned to the test device again. Specifically, the insertion element 2017 of the collector is inserted into the jack 5014 of the accommodating chamber 504. For the ease of the insertion, the entire collector is generally inserted into the accommodating chamber 504 obliquely. When the insertion element 2017 of the collector is inserted into the jack 5014, a front end of the absorption element is in contact with a part of the sample application area located at the bottom of the accommodating chamber 504. Then, the handle 201 is allowed to enter the docking area 505, the handle hand-held area 90 is allowed to be located at the place indicated by the docking area 506, the protruding structure 903 is allowed to be clamped in the bayonet 507, and the handle is allowed to be fixed onto the docking area. When the handle obliquely enters the docking area and is fixed onto the docking area, the carrier 206 completely covers the accommodating chamber, and the absorption element on the carrier completely covers the sample application area at the bottom of the accommodating chamber, such that the absorption element is compressed to release the liquid sample to the sample application area for testing the analyte in the liquid sample, and the testing result is displayed in the testing area, and can be observed through the window area, for example, whether a T line appears and whether a C line appears are observed to identify the effectiveness of the testing result. If an overall thickness formed by the absorption element and the carrier (the absorption element is not compressed) is greater than a distance between the jack and the sample application area of the testing element at the bottom of the accommodating chamber, the absorption element is allowed to be compressed during such an operation. Specific examples are as follows: generally, a distance H1 from the sample application area to the jack is about 1-2 mm; a thickness h2 of the absorption element is about 2-5 mm, for example, 3 mm; a thickness h3 of the carrier is about 2-4 mm, for example, 2 mm; and a sum of the thickness of the absorption element and the thickness of the carrier is 5 mm. If there is no any force applied to the carrier and when the absorption element is arranged inside the accommodating chamber, the thickness h2 of the absorption element is greater than the distance H1 from the sample application area to the jack, and the depth H3 of the docking area is about 2-3 mm, for example, 3 mm (also greater than H1). In such arrangement, when the handle enters into the docking area and is fixed to the test device, the handle drives the carrier 206 to move downward and applies a force to the carrier 206, the carrier naturally extrudes the absorption element, thereby allowing the absorption element to be compressed by 1 -2 millimeters. The sum of the thickness of the carrier and the thickness of the absorption element (h2 + h3) is at least less than or equal to H1, and the distance change is precisely caused by thickness decrease as a result of the absorption element being compressed. Generally, the thickness of the carrier is unchanged, which can be easy to realize that the absorption element is compressed to release the liquid samples to the sample application area of the testing element. This can explain why, during the insertion of the collector into the accommodating chamber and the entry of the handle into the docking area, the absorption element can be extruded without the continued application of pressure by the sliding pressing element in the previous embodiment example, the operation completes the detection in just one step without the need for the sliding pressing element 500.

In another aspect of the present invention, a novel collector is provided, as shown in FIG. 9, and FIG. 11 - FIG. 13. The collector includes an absorption element 207 for absorbing a liquid sample. The absorption element is fixed to the carrier 206 of the absorption head, and the carrier may be made of rigid plastics or any other materials, generally not compressible, and preferably made of transparent materials. One side of the carrier 206 is provided with the absorption element 207 and the other side thereof covers the frame body 205, such that a chamber 216 is formed on one end of the collector. The indicator element 208 is arranged in the chamber and connected with the absorption element 207, thereby indicating whether the absorption element has collected full or sufficient samples. Alternatively, the carrier is provided with a groove 217 in which the indicator element 208 is arranged; and when the absorption element covers the carrier, the indicator element directly covers the back face of the absorption element. In some embodiments, the carrier turns transparent green when contacting water, and the indicator element 208 turns from white or colorless to red or burgundy when contacting water. Therefore, whether the absorption element 207 absorbs enough liquid samples can be identified with naked eyes. The frame body 205 and the carrier 206 are both made of relatively rigid materials, and the absorption element can be compressed; a pressure being applied to the frame body 205 can cause the absorption element 207 to be compressed, thereby releasing liquid, for example, releasing liquid onto the sample application area of the testing element at the bottom of the accommodating chamber.

Such collector is particularly suitable for sampling saliva from the oral cavity, and the collector with the absorption element 207 extends into the oral cavity and keeps it here for a short time to collect a saliva sample. When the indicator element 208 turns color, the saliva sample is sufficient and a subsequent test can be conducted. In some embodiments, the tail end of the collector is further provided with an insertion element 206 inserted into the jack the accommodating chamber 302. After the collector collects the liquid sample, the insertion element 206 is inserted into the jack on one end of the accommodating chamber 302, such that the collector can be kept at the fixed position of the accommodating chamber 302, namely a bottom of the accommodating chamber 302 and the sample application area of the testing element, thereby avoiding inaccurate testing results caused by damage to the sample application area or change in the physical position of the sample application area when the absorption element is extruded or inserted.

This specification also includes the subject matter of the following clauses
1. A test device, comprising a detection chamber, wherein the detection chamber comprises a testing element therein, and the testing element is configured to test an analyte in a liquid sample;
   the test device further comprising a sample collector, wherein the sample collector comprises an absorption element, the absorption element is configured to absorb the liquid sample, an accommodating chamber is provided on or in the test device, and the accommodating chamber is configured to accommodate the absorption element of the sample collector. The test device according to clause 1, a part of the absorption element is located in the accommodating chamber directly or indirectly or the whole of the absorption element is located in the accommodating chamber directly or indirectly.
2. The test device according to clauses 1, wherein the testing element comprises a testing area and a sample application area, and a part of the sample application area is located in the accommodating chamber.
3. The test device according to clause 2, wherein the absorption element of the sample collector has a first state and a second state in the accommodating chamber; and when the absorption element of the sample collector being in the first state, the absorption element is not in direct contact with the sample application area.
4. The test device according to clause 3, wherein the absorption element of the sample collector is in the second state, the absorption element is in direct contact with the sample application area or pressed on the sample application area of the test element.
5. The test device according to clause 4, wherein with the absorption element of the sample collector being in the second state, the absorption element absorbs the liquid sample and is compressed, thereby releasing the liquid sample onto the sample application area.
6. The test device according to clause 5, wherein the compression means that when the absorption element of the sample collector enters into the accommodating chamber and is in the second state or a process of entering into the accommodating chamber, the absorption element interacts with the accommodating chamber to generate a pressure, and the pressure causes the absorption element to be compressed.
7. The test device according to clause 6, wherein the sample collector comprises an insertion element, and the accommodating chamber comprises a jack; and when the insertion element is inserted into the jack or in an insertion process, the absorption element interacts with and the accommodating chamber.
8. The test device according to clause 6, wherein the sample collector comprises a carrier, the absorption element is loaded on the carrier, the carrier comprises the insertion element, and the insertion element is configured to be inserted into the jack in the accommodating chamber; and when the absorption element is disposed in the accommodating chamber and is in direct contact with the sample application area, the insertion element is inserted into the jack such that the carrier applies the pressure to the absorption element.
9. The test device according to clause 8, wherein the absorption element is fixed onto the carrier and the carrier comprises an indicator strip that is in fluid communication with the absorption element and configured to indicate whether the absorption element has fully absorbed a liquid sample.
10. The test device according to clause 8, wherein the sample collector further comprises a handle integrally connected with the carrier, and the test device further comprises a docking area for accommodating the handle of the sample collector.
11. The test device according to clause 10, wherein when the absorption element is disposed in the accommodating chamber and in direct contact with the sample application area, the handle of the sample collector is fixed in the docking area of the test device; and when the handle is in a process of entering the docking area, the handle applies the pressure to the carrier, and the pressure causes the absorption element to be compressed.
12. The test device according to clause 10, further comprising a slidable pressure element, wherein the pressure element is capable to slide onto the accommodating chamber to apply the pressure to the carrier and whereby compress the absorption element.
13. The test device according to clause 11, further comprising a window area for reading a testing result located downstream of the accommodating chamber, wherein the pressure element is located on the window area and hides the window area before sliding to the accommodating chamber.
14. The test device according to clause 13, wherein the pressure element is capable to slide from the window area onto the accommodating chamber to expose the window area.
15. The test device according to clause 12, wherein the docking area for accommodating the handle is located upstream of the accommodating chamber.
16. The test device according to clause 3, wherein with the absorption element of the sample collector being in the first state, the sample collector does not absorb the liquid sample.
17. The test device according to clause 3, wherein with the absorption element of the sample collector being in the first state, the absorption element is protected by a cover body, and a part of the cover body is located in the accommodating chamber.
18. The test device according to clause 3, wherein with the absorption element of the sample collector being in the first state, a pressure element is located on a window area, through which for viewing a test result on the test area of the test element.
19. The test device according to clause 12, wherein the pressure element comprises two clamping parts, a sliding rail or a chute is provided on the clamping parts, a sliding rail and a chute are provided on two longitudinal sides of the test device, the sliding rail or the chute on the clamping parts fits with the chute or the sliding rail on the test device, such that the pressure element is capable to move longitudinally along the test device.
20. The test device according to clause 19, wherein the testing element further comprises a label area located downstream of the sample application area and a testing area located downstream of the label area, and the testing area is located in the window area.
21. The test device according to clause 20, wherein no any label is present in the sample application area.
22. A method for testing an analyte in a liquid sample, comprising:
   providing a test device, wherein the test device comprises a detection chamber comprising a testing element, and the testing element is configured to test the analyte in the liquid sample; and a sample collector, wherein the sample collector comprises an absorption element configured to absorb the liquid sample, an accommodating chamber is provided on the test device, and the accommodating chamber comprises the absorption element; and wherein the accommodating chamber comprises a sample application area of the testing element;
   the absorption element is allowed to have a first state and a second state in the accommodating chamber; and when the absorption element of the sample collector is in the first state, the absorption element is not allowed to be in direct contact with the sample application area.
23. The method according to clause 22, comprising
   allowing the absorption element of the sample collector to depart from the accommodating chamber of the test device; allowing the absorption element to absorb the liquid sample and then enter the accommodating chamber of the test device; and allowing the absorption element to be in direct contact with the sample application area of the testing element.
24. The method according to clause 19, wherein when the absorption element is in contact with the sample application area, an insertion element on the sample collector interacts with a jack of the accommodating chamber to generate a pressure, and the pressure causes the absorption element to be compressed and causes the liquid sample to be released into the sample application area.
25. The method according to clause 24, wherein the sample collector comprises a handle and a carrier connected with the handle, the absorption element is loaded on the carrier, and the carrier comprises the insertion element, such that the insertion element is obliquely inserted into the jack of the accommodating chamber, and the handle is also inclined at an angle.
26. The method according to clause 25, wherein the handle of the sample collector is allowed to be changed from the inclined angle to a position parallel to the test device, such that the carrier is covered in the accommodating chamber with the change of the handle, a pressure is applied to the carrier, and the pressure squeezes the absorption element and releases the liquid sample.
27. The method according to clause 26, wherein the test device further comprises a docking area for accommodating the handle of the sample collector; and when the handle is parallel to the test device, the handle is located in the docking area, and the docking area is located upstream of the accommodating chamber.
28. The method according to clause 24, wherein the test device further comprises a pressure element capable to slide longitudinally, and the pressure element covers a window area for reading a testing result of the test device; and after the absorption element is compressed in the accommodating chamber, the pressure element is allowed to slide from the window area to the accommodating chamber to continuously apply pressure to the absorption element.
29. The method according to clause 22, wherein the absorption element further comprises a cover body, and the cover body is located in the accommodating chamber, such that the absorption element is in the first state without being in direct contact with the sample application area.
30. The method according to clause 25, wherein the absorption element is located on the carrier, and the carrier comprises an indicator strip that is in fluid communication with the absorption element; and when the absorption element absorbs the liquid sample, the indicator strip is used for indicating whether the absorption element has fully absorbed the liquid sample.
31. The method according to clause 29, wherein the carrier and the absorption element located on the carrier are allowed to be located in the cover body.
32. The method according to clause 22, wherein the liquid sample is a saliva sample, and the absorption element is allowed to extend into the mouth to collect the saliva sample.
33. A liquid sample collector, comprising a handle and a rigid carrier connected with the handle, wherein the rigid carrier comprises an absorption element capable to be compressed, and the absorption element is configured to absorb a liquid sample; wherein the carrier is transparent, and the carrier comprises an indicator strip that is in fluid communication with the absorption element; and the indicator strip is used for indicating whether the absorption element has fully absorbed the liquid sample.
34. The collector according to clause 33, wherein the carrier is located on a window structure, the carrier is provided with a groove, and the indicator strip is located in the groove and directly covers the absorption element.
35. The collector according to clause 33, wherein the collector further comprises an insertion element, and the insertion element is located on the carrier and configured to be inserted into a jack of an accommodating chamber.
36. The collector according to clause 33, wherein the indicator strip displays red or pink when contacting liquid, and is colorless or white when being dry.

All the patents and publications mentioned in the description of the present invention indicate that these are public technologies in the art and can be used by the present invention. All the patents and publications cited herein are listed in the references, just as each publication is specifically referenced separately. The present invention described herein can be realized in the absence of any one element or multiple elements, one restriction or multiple restrictions, where such restriction is not specifically described here. For example, the terms "comprising", "essentially consisting of" and "consisting of" in each embodiment herein may be replaced by the rest 2 terms. The so-called "alan" herein merely means "one", but does not exclude including 2 or more instead of including only one. The terms and expressions which have been employed herein are descriptive rather than restrictive, and there is no intention to suggest that these terms and expressions in this description exclude any equivalents, but it is to be understood that any appropriate changes or modifications can be made within the scope of the present invention and appended claims. It can be understood that the embodiments described in the present invention are some preferred embodiments and features. A person skilled in the art can make some modifications and changes according to the essence of the description of the present invention. These modifications and changes are also considered to fall within the scope of the present invention and the scope limited by independent claims and dependent claims.

## Claims

1. A test device, comprising a detection chamber, wherein the detection chamber comprises a testing element therein, and the testing element is configured to test an analyte in a liquid sample; the test device further comprising a sample collector, wherein the sample collector comprises an absorption element, the absorption element is configured to absorb the liquid sample, an accommodating chamber is provided on or in the test device, and the accommodating chamber is configured to accommodate the absorption element of the sample collector.

2. The test device according to claim 1, wherein the testing element comprises a testing area and a sample application area, and a part of the sample application area is located in the accommodating chamber.

3. The test device according to one of claims 1-2, wherein the absorption element of the sample collector has a first state and a second state in the accommodating chamber; and when the absorption element of the sample collector is in the first state, the absorption element is not in direct contact with the sample application area.

4. The test device according to claim 3, wherein when the absorption element of the sample collector is in the second state, the absorption element is in direct contact with the sample application area or pressed on the sample application area of the test element.

5. The test device according to one of claims 3-4, wherein when the absorption element of the sample collector is in the second state, the absorption element comprises the liquid sample and is compressed so as to release the liquid sample onto the sample application area.

6. The test device according to one of claims 3-5, wherein when the absorption element of the sample collector enters into the accommodating chamber, is in the second state or a process of entering into the accommodating chamber, the absorption element interacts with the accommodating chamber to generate a pressure, and the pressure causes the absorption element to be compressed.

7. The test device according to one of claims 1-6, wherein the sample collector comprises an insertion element, and the accommodating chamber comprises a jack; and when the insertion element is inserted into the jack or in an insertion process, the absorption element interacts with the accommodating chamber to generate a pressure.

8. The test device according to claim 7, wherein the sample collector comprises a carrier, the absorption element is loaded on the carrier, the carrier comprises the insertion element, and the insertion element is configured to be inserted into the jack of the accommodating chamber; and when the absorption element is disposed in the accommodating chamber and is in direct contact with the sample application area, the insertion element is inserted into the jack such that the carrier applies the pressure to the absorption element.

9. The test device according to one of claims 1- 8, wherein the absorption element is fixed onto the carrier and the carrier comprises an indicator strip that is in fluid communication with the absorption element and configured to indicate whether the absorption element has fully absorbed a liquid sample.

10. The test device according to one of claims 1-9, wherein the sample collector further comprises a handle integrally connected with the carrier, and the test device further comprises a docking area for accommodating the handle of the sample collector.

11. The test device according to claim 10, wherein when the absorption element is disposed in the accommodating chamber and in direct contact with the sample application area, the handle of the sample collector is fixed in the docking area of the test device; and when the handle is in a process of entering the docking area, the handle applies a pressure to the absorption element or the carrier, and the pressure causes the absorption element to be compressed.

12. The test device according to one of claims 1-10, further comprising a pressure element, wherein the pressure element is capable to slide or turn onto the accommodating chamber to apply a pressure to the absorption element or the carrier and whereby compress the absorption element.

13. The test device according to claim 12, further comprising a window area for reading a testing result located downstream of the accommodating chamber, wherein the pressure element is located on the window area and hides the window area before sliding or turning to the accommodating chamber.

14. The test device according to claim 13, wherein the pressure element is capable to slide or be turned over from the window area onto the accommodating chamber to expose the window area.

15. The test device according to claim 3, wherein with the absorption element of the sample collector being in the first state, the sample collector does not absorb the liquid sample.
